# EUROPEAN PATENT APPLICATION

(11) **EP 4 212 156 A1**
(43) Date of publication of application: **19.07.2023**
(21) Application number: 22305029.5
(22) Date of filing: 13.01.2022
(51) Int. Cl.: A61K 31/405, A61K 31/47, A61K 31/706, A61P 1/00, A61P 9/00, A61P 11/00, A61P 13/00, A61P 19/00, A61P 21/00, A61P 25/00, A61P 27/00, A61P 29/00, A61P 31/00

(54) **COMBINATION OF 8-CHLORO-N-(4-(TRIFLUOROMETHOXY)PHENYL)QUINOLIN-2-AMINE AND ITS DERIVATIVES WITH A S1P RECEPTOR MODULATOR**

(71) Applicant: ABIVAX, 75008 Paris (FR)
(72) Inventor: The designation of the inventor has not yet been filed
(74) Representative: Cabinet Nony

(57) **Abstract**

The present invention relates to a pharmaceutical combination of a compound of formula (I) or a pharmaceutically acceptable salt thereof, prodrug thereof or a metabolite thereof with a S1P receptor modulator or a pharmaceutically acceptable salt thereof, wherein the compound of formula (I) has the following formula:

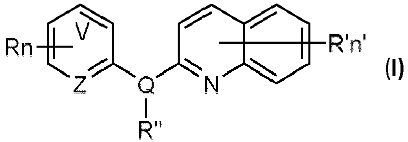

It further relates to a pharmaceutical composition comprising said combination, its use and a pharmaceutical kit comprising it.

## Description

### FIELD OF THE INVENTION

This invention relates to a pharmaceutical composition comprising 8-Chloro-N-(4-(trifluoromethoxy)phenyl)quinolin-2-amine, one of its prodrug or derivatives, or one of their pharmaceutically acceptable salt or metabolite thereof and a S1P receptor modulator or a pharmaceutically acceptable salt thereof.

The present invention provides a combination of 8-Chloro-N-(4-(trifluoromethoxy)phenyl)quinolin-2-amine, one of its prodrug or derivatives, or one of their pharmaceutically acceptable salt or metabolite thereof, and particularly of its glucuronide metabolite with a S1P receptor modulator, or pharmaceutically acceptable salt thereof, which may be particularly useful in the treatment of inflammatory diseases, even more particularly of inflammatory bowel disease, including ulcerative colitis and Crohn's disease, rheumatoid arthritis and dermatitis.

### BACKGROUND OF THE INVENTION

Some quinoline derivatives have been described in WO2010/143169, WO2012/080953, WO2016/009065, WO2016/009066 and WO2020/127843, the contents of which are incorporated herein by reference in their entireties.

8-Chloro-N-(4-(trifluoromethoxy)phenyl)quinolin-2-amine or one of its pharmaceutically acceptable salt was among all disclosed is such patents applications as well as its use in the treatment of inflammatory diseases. Its glucuronide metabolite was in particular disclosed in WO 2016/135052.

The sphingosine-1-phosphate receptors are a class of G protein-coupled receptors that are targets of the lipid signalling molecule Sphingosine-1-phosphate (S1P). They are divided into five subtypes: S1PR1, S1PR2, S1PR3, S1PR4 and S1PR5.

S1P has been implicated in a variety of diseases such as cancer, degenerative diseases such as Alzheimer's disease, sickle cell disease and fibrosis. S1P receptor modulators, comprising non-selective S1P receptor modulators and selective S1P receptor modulators, are also named sphingosine-1-phosphate receptor 1 modulators and act as functional antagonists on the S1P receptor.

S1P receptor modulators have been described as being useful for the prevention and/or treatment of diseases or disorders associated with an activated immune system. More recently, they have been described as being useful in the treatment of inflammatory diseases such as multiple sclerosis, rheumatoid arthritis, skin inflammation, neuroinflammation and inflammatory bowel disease, including Crohn's disease and Ulcerative Colitis.

Some side-effects have been revealed in the literature when using S1P receptor modulator such as a risk for bradyarrhythmia and atrioventricular blocks (AV blocks), but also risk of lymphopenia, and in certain cases severe lymphopenia, potentially associated with an increased risk of infection.

None of the hereabove cited documents disclose a synergistic activity of a combination of compounds of formula (I) as defined herein after, in particular 8-Chloro-N-(4-(trifluoromethoxy)phenyl)quinolin-2-amine, or one of their pharmaceutically acceptable salt or metabolite thereof with a S1P receptor modulator nor the advantage of this combination for decreasing the hereabove mentioned side-effects.

The mechanisms of action and side effects of S1P receptor modulators and being different from the one of 8-Chloro-N-(4-(trifluoromethoxy)phenyl)quinolin-2-amine, assessing synergistic efficacy on inflammation is of interest.

There is still a need for alternative or complementary therapies efficient in the treatment of inflammatory diseases, disorders or conditions such as inflammatory bowel disease.

There also remains a need for compounds with no or limited side-effects.

### SUMMARY OF THE INVENTION

It has now been found that a combination of a compound of formula (I) as defined herein after, a pharmaceutically acceptable salt thereof, prodrug or a metabolite thereof, in particular 8-Chloro-N-(4-(trifluoromethoxy)phenyl)quinolin-2-amine, with a S1P receptor modulator, or pharmaceutically acceptable salt thereof is useful for the treatment and/or prevention of a variety of inflammatory diseases, disorders or conditions.

In particular, the invention relates to compounds to be used to reduce the dose of a S1P receptor modulator, or pharmaceutically acceptable salt thereof and thereby limiting its side effects, while preserving or increasing the said efficacy and/or safety to treat said variety of inflammatory diseases, disorders or conditions, as detailed herein after of said S1P receptor modulator, or pharmaceutically acceptable salt thereof.

In particular, the invention relates to compounds to be used to reduce the dose of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt, prodrug or metabolite thereof, and particularly of its glucuronide metabolite and thereby limiting its side effects, while preserving or increasing the said efficacy and/or safety to treat said variety of inflammatory diseases, disorders or conditions, as detailed herein after of said compound of formula (I), or pharmaceutically acceptable salt, prodrug or metabolite thereof.

Herein is provided a combination therapy for treating inflammatory diseases, disorders or conditions as detailed herein after and in particular inflammatory bowel disease, including ulcerative colitis and of Crohn's disease, rheumatoid arthritis and dermatitis.

It is more particularly herein provided a pharmaceutical combination of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt, prodrug or metabolite thereof, and particularly of its glucuronide metabolite, with a S1P receptor modulator or a pharmaceutically acceptable salt thereof.

Herein is also provided a pharmaceutical combination of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, with a S1P receptor modulator or a pharmaceutically acceptable salt thereof for use in the treatment of inflammatory diseases, disorders or conditions as detailed herein after and in particular inflammatory bowel disease, including ulcerative colitis and of Crohn's disease, rheumatoid arthritis and dermatitis.

The compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, and the S1P receptor modulator or a pharmaceutically acceptable salt thereof may be used simultaneously, separately or may be spread out over time, and preferably simultaneously.

Accordingly, herein is provided a pharmaceutical combination of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, and of a S1P receptor modulator or a pharmaceutically acceptable salt thereof for separate administration, administration spread out over time or simultaneous administration to patients suffering from inflammatory diseases, disorders or conditions as detailed herein after and in particular inflammatory bowel disease, including ulcerative colitis and of Crohn's disease, rheumatoid arthritis and dermatitis.

Herein is further provided a pharmaceutical composition comprising a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite with a S1P receptor modulator or a pharmaceutically acceptable salt thereof and at least one pharmaceutically acceptable excipient.

Herein is further provided a pharmaceutical composition comprising 8-Chloro-N-(4-(trifluoromethoxy)phenyl)quinolin-2-amine or one of its pharmaceutically acceptable salts and etrasimod or a pharmaceutically acceptable salt thereof and at least one pharmaceutically acceptable excipient.

Herein is further provided a pharmaceutical composition comprising 8-Chloro-N-(4-(trifluoromethoxy)phenyl)quinolin-2-amine glucuronide metabolite and etrasimod or a pharmaceutically acceptable salt thereof and at least one pharmaceutically acceptable excipient.

The pharmaceutical combination comprises an effective amount, as implemented in the present combination, of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salts or one of its metabolite and an effective amount, as implemented in the present combination, of a S1P receptor modulator or a pharmaceutically acceptable salt thereof.

Herein is further provided a compound of formula (I) as defined herein after, a pharmaceutically acceptable salt thereof, prodrug thereof or a metabolite thereof, in particular 8-Chloro-N-(4-(trifluoromethoxy)phenyl)quinoline-2-amine or one of its pharmaceutically acceptable salt or metabolite thereof, and particularly of its glucuronide metabolite for increasing the efficacy and/or safety of a S1P receptor modulator or a pharmaceutically acceptable salt thereof, in particular for its anti-inflammatory activity, and hereby reducing the S1P receptor modulator dose while maintaining its efficacy, in a subject suffering from inflammatory diseases, disorders or conditions as detailed herein after, and in particular inflammatory bowel disease, including ulcerative colitis and of Crohn's disease rheumatoid arthritis and dermatitis, in need thereof.

Herein is further provided a S1P receptor modulator or a pharmaceutically acceptable salt thereof for increasing the efficacy and/or safety of a compound of formula (I) as defined herein after, a pharmaceutically acceptable salt thereof, prodrug thereof or a metabolite thereof, in particular 8-Chloro-N-(4-(trifluoromethoxy)phenyl)quinoline-2-amine or one of its pharmaceutically acceptable salt or metabolite thereof, and particularly of its glucuronide metabolite, in particular for its anti-inflammatory activity, and hereby reducing the compound of formula (I) or pharmaceutically acceptable salt, prodrug or metabolite thereof or dose while maintaining its efficacy, in a subject suffering from inflammatory diseases, disorders or conditions as detailed herein after, and in particular inflammatory bowel disease, including ulcerative colitis and of Crohn's disease rheumatoid arthritis and dermatitis, in need thereof.

Herein is further provided a pharmaceutical kit, in particular intended for treating inflammatory diseases, disorders or conditions as detailed herein after, and in particular inflammatory bowel disease, including ulcerative colitis and of Crohn's disease, rheumatoid arthritis and dermatitis, comprising:
(i) a first galenical formulation comprising a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, and
(ii) a second galenical formulation comprising a S1P receptor modulator or a pharmaceutically acceptable salt thereof.

In the framework of the present invention, the following definitions may be given:
- effective amount: amount of a pharmaceutical compound which produces an effect on the disease treated;
- the separate administration, simultaneous administration or administration spread out over time of a medicinal combination means that the elementary constituents of the combination, can be administered at the same time, each in one go at distinct moments, or repeatedly, or else at different moments, in particular during cycles. The elementary constituents can, in order to do this, be formulated as mixtures, only if they are administered simultaneously, or else formulated separately for the other administration schemes;
- As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio; and
- The term "patient" as used herein, means an animal, preferably a mammal, and most preferably a human.
- As used herein, the term "side effects" has its general meaning in the art and refers to unintended effect occurring at normal dose related to the pharmacological properties.

In the framework of the present invention, for a sake of simplification, the term "a combination according to the present invention" or "a pharmaceutical combination" means a pharmaceutical combination of a compound of formula (I) as defined herein after, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, with a S1P receptor modulator or one of its pharmaceutically acceptable salt.

Synergy or synergistic effect of a combination of drugs means that the combined effect is greater than that predicted by the individual potencies of said drugs. A synergistic interaction may allow a greater therapeutic efficacy, and/or the use of lower doses of said drugs, for example of at least one of them, and may reduce adverse reactions or side effects.

As a way of illustration, assessment of drug combinations synergy is discussed in Genes Cancer. 2011 Nov; 2(11): 1003-1008 (doi: 10.1177/1947601912440575). It is among all stated that "*If the combined effect observed is significantly greater than the expected (additive) effect, there is synergism".* Said assessment of drug combinations synergy in the framework of the present invention is not limited to the use of a unique method but rather can be based on the use of any method well known to the man skilled in the art.

### DETAILED DESCRIPTION OF THE INVENTION

The compound of formula (I) that may be implemented in the framework of the present invention may be represented herein after: 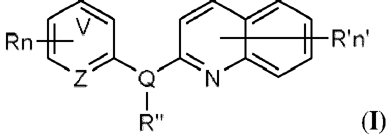 or a pharmaceutically acceptable salt thereof or prodrug thereof,
wherein:
Z is C or N;
V is C or N; 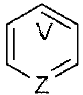 means an aromatic ring wherein V is C or N, and when V is N, V is ortho, meta or para relative to Z;
each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R₁, -NR₁-C(=O)-R₁, -NR₁-C(=O)-NR₁R₂, -SO₂₋NR₁R₂, -SO₃H, -O-SO₂-OR₃, -O-P(=O)-(OR₃)(OR₄), -O-CH₂-COOR₃, (C₁-C₃)alkyl, said alkyl being optionally mono- or di-substituted by a hydroxyl group, a group of formula (IIa): 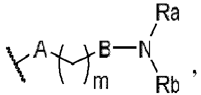
or a group of formula 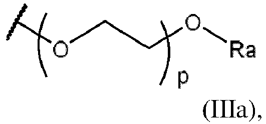
Q is N or O, provided that R" does not exist when Q is O;
each of R₁ and R₂ is independently hydrogen or (C₁-C₃)alkyl;
each of R₃ and R₄ is independently hydrogen, Li⁺, Na⁺, K⁺, N⁺(Ra)₄
or benzyl; n is 1, 2 or 3;
n' is 1, 2 or 3;
each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy,-O-P(=O)-(OR₃)(OR₄),- CN,
a group of formula (IIa): 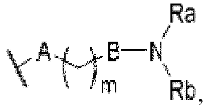
or a group of formula (IIIa): 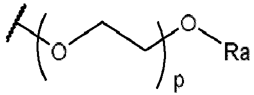
A is a covalent bond, oxygen, or NH;
B is a covalent bond or NH;
m is 1, 2, 3, 4 or 5;
p is 1, 2 or 3; each of Ra and Rb is independently hydrogen, (C₁-C₅)alkyl, or (C₃₋C₆)cycloalkyl, or
Ra and Rb form together with the nitrogen atom to which they are attached a saturated 5- or 6- membered heterocycle, said heterocycle being optionally substituted by one or more Ra, provided that when R' is a group (IIa) or (IIIa), n' may be 2 or 3 only if other R' groups are different from said group (IIa) or (IIIa); and
R" is hydrogen, (C₁-C₄)alkyl, or a group of formula (IIa) as defined herein.

The compound of formula (I) or salts thereof may form solvates or hydrates and the invention includes all such solvates and hydrates. The terms "hydrates" and "solvates" simply mean that the compounds according to the invention can be in the form of a hydrate or solvate, i.e. combined or associated with one or more water or solvent molecules. This is only a chemical characteristic of such compounds, which can be applied for all organic compounds of this type.

The compound of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

In the context of the present invention, the term:
- "halogen" is understood to mean chlorine, fluorine, bromine, or iodine, and in particular denotes chlorine, fluorine or bromine,
- "(C₁-C₅)alkyl" as used herein respectively refers to C₁-C₅ normal, secondary or tertiary saturated hydrocarbon. Examples are, but are not limited to, methyl, ethyl, 1-propyl, 2-propyl, butyl, pentyl,
- "(C₃-C₆)cycloalkyl" as used herein respectively refers to cyclic saturated hydrocarbon.

Examples are, but are not limited to cyclopropyl, cyclobutyl, cyclopentyl, cyclohexyl,
- "(C₁-C₄)alkoxy" as used herein respectively refers to O-(C₁-C₄)alkyl moiety, wherein alkyl is as defined above. Examples are, but are not limited to, methoxy, ethoxy, 1-propoxy, 2-propoxy, butoxy,
- "fluoroalkyl group" and "fluoroalkoxy group" refers respectively to alkyl group and alkoxy group as above-defined, said groups being substituted by at least one fluorine atom. Examples are perfluoroalkyl groups, such as trifluoromethyl or perfluoropropyl,
- "saturated 5- or 6-membered heterocycle" as used herein respectively refers to a saturated cycle comprising at least one heteroatom. Examples are, but are not limited to, morpholine, piperazine, thiomorpholine, piperidine, pyrrolidine.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

As defined generally above, Z is C or N.

In some embodiments, Z is C. In some embodiments, Z is N.

In some embodiments, Z is selected from those depicted in **Tables 1-3,** below.

As defined generally above, V is C or N.

In some embodiments, V is C. In some embodiments, V is N.

In some embodiments, V is selected from those depicted in **Tables 1-3,** below.

As defined generally above, 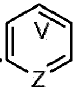 means an aromatic ring wherein V is C or N and when V is N, V is ortho, meta or para relative to Z.

In some embodiments, 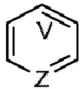 means an aromatic ring wherein V is C.

In some embodiments, 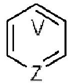 means an aromatic ring wherein V is N, and V is ortho, meta or para relative to Z.

In some embodiments, V is N, and V is ortho relative to Z. In some embodiments, V is N, and V is meta relative to Z. In some embodiments, V is N, and V is para relative to Z.

In some embodiments, 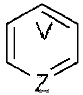 is phenyl.

In some embodiments, 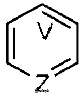 is pyridine.

In some embodiments, 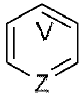 is pyridazine.

In some embodiments, 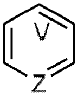 is pyrimidine.

In some embodiments, 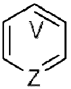 is pyrazine.

In some embodiments, below. 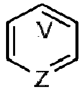 is selected from those depicted in **Tables 1-3,**

As described generally above, each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy,phenoxy, -NR₁-SO₂-NR₁R₂, -NR₁-SO₂-R₁, -NR₁-C(=O)-R₁, -NR₁₋C(=O)-NR₁R₂, -SO₂-NR₁R₂, -SO₃H, -O-SO₂-OR₃, -O-P(=O)-(OR₃)(OR₄), -O-CH₂-COOR₃, (C₁-C₃)alkyl, said alkyl being optionally mono- or di-substituted by a hydroxyl group or a group of formula (IIa): 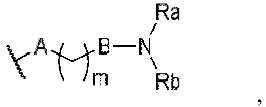 or a group of formula (IIIa):

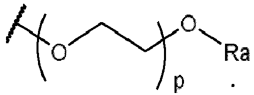

In some embodiments, R is hydrogen. In some embodiments, R is halogen. In some embodiments, R is -CN. In some embodiments, R is hydroxyl. In some embodiments, R is (C₁-C₃)fluoroalkyl, said alkyl being optionally mono- or di- substituted by hydroxyl. In some embodiments, R is (C₁-C₃)fluoroalkoxy. In some embodiments, R is (C₃-C₆)cycloalkyl. In some embodiments, R is -NO₂. In some embodiments, R is -NR₁R₂. In some embodiments, R is (C₁-C₄)alkoxy. In some embodiments, R is phenoxy. In some embodiments, R is -NR₁-SO₂-NR₁R₂. In some embodiments, R is - NR₁-SO₂-R₁. In some embodiments, R is -NR₁-C(=O)-R₁. In some embodiments, R is -NR₁-C(=O)-NR₁R₂. In some embodiments, R is -SO₂-NR₁R₂. In some embodiments, R is -SO₃H. In some embodiments, R is -O-SO₂-OR₃. In some embodiments, R is -O-P(=O)-(OR₃)(OR₄). In some embodiments, R is -O-CH₂-COOR₃. In some embodiments, R is (C₁-C₃)alkyl, said alkyl being optionally mono- or di- substituted by hydroxyl.

In some embodiments, each R is independently halogen, (C₁-C₃)fluoroalkyl, (C₁-C₃)fluoroalkoxy, -NR₁R₂, (C₁-C₄)alkoxy, or (C₁-C₃)alkyl.

In some embodiments, each R is independently hydrogen, methyl, methoxy, trifluoromethyl, trifluoromethoxy, amino, halogen, or -O-P(=O)-(OR₃)(OR₄). In some embodiments, R is methyl. In some embodiments, R is methoxy. In some embodiments, R is trifluoromethyl. In some embodiments, R is trifluoromethoxy. In some embodiments, R is amino. In some embodiments, R is -O-P(=O)-(OR₃)(OR₄).

In some embodiments, each R is independently methyl, methoxy, trifluoromethyl, halogen, trifluoromethoxy, or amino.

In some embodiments, R is selected from those depicted in **Tables 1-3,** below.

As described generally above, Q is N or O, provided that R" does not exist when Q is O.

In some embodiments, Q is N. In some embodiments, Q is O, and R" does not exist.

In some embodiments, Q is selected from those depicted in **Tables 1-3,** below.

As described generally above, each of R₁ and R₂ is independently hydrogen or (C₁-C₃)alkyl.

In some embodiments, R₁ is hydrogen. In some embodiments, R₁ is (C₁-C₃)alkyl. In some embodiments, R₂ is hydrogen. In some embodiments, R₂ is (C₁-C₃)alkyl.

In some embodiments, each of R₁ and R₂ is independently selected from those depicted in **Tables 1-3,** below.

As described generally above, each of R₃ and R₄ is independently hydrogen, Li⁺, Na⁺,K⁺, N⁺(Ra)₄ or benzyl.

In some embodiments, R₃ is hydrogen. In some embodiments, R₃ is Li⁺. In some embodiments, R₃ is Na⁺. In some embodiments, R₃ is K⁺. In some embodiments, R₃ is N⁺(Ra)₄. In some embodiments, R₃ is benzyl. In some embodiments, R₄ is hydrogen. In some embodiments, R₄ is Li⁺. In some embodiments, R₄ is Na⁺. In some embodiments, R₄ is K⁺. In some embodiments, R₄ is N⁺(Ra)₄. In some embodiments, R₄ is benzyl.

In some embodiments, each of R3 and R4 is independently selected from those depicted in **Tables 1-3,** below.

As described generally above, n is 1, 2 or 3.

In some embodiments, n is 1 or 2. In some embodiments, n is 1. In some embodiments, n is 2. In some embodiments, n is 3.

In some embodiments, n is selected from those depicted in **Tables 1-3,** below.

As described generally above, n' is 1, 2 or 3.

In some embodiments, n' is 1 or 2. In some embodiments, n' is 1. In some embodiments, n' is 2. In some embodiments, n' is 3.

In some embodiments, n' is selected from those depicted in **Tables 1-3,** below. As described generally above, each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy, -O-P(=O)-(OR₃)(OR₄), -CN, a group of formula (IIa): 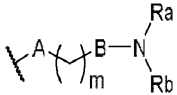 , or a group of formula (IIIa):

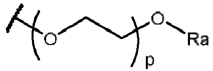

In some embodiments, R' is hydrogen. In some embodiments, R' is (C₁-C₃)alkyl. In some embodiments, R' is Hydroxyl. In some embodiments, R' is halogen. In some embodiments, R' is -NO₂. In some embodiments, R' is -NR₁R₂. In some embodiments, R' is morpholinyl. In some embodiments, R' is morpholino. In some embodiments, R' is N-methylpiperazinyl. In some embodiments, R' is (C₁-C₃)fluoroalkyl. In some embodiments, R' is (C₁-C₄)alkoxy. In some embodiments, R' is -O-P(=O)-(OR₃)(OR₄). In some embodiments, R' is -CN.

In some embodiments, R' is a group of formula (IIa):

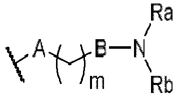

In some embodiments, R' is a group of formula (IIIa):

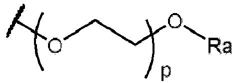

In some embodiments, R' is amino. In some embodiments, R' is methyl. In some embodiments, R' is a group of formula 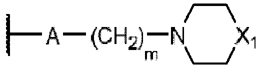 wherein A is O or NH, m is 2 or 3 and X₁ is O, CH₂ or N-CH₃, provided that when R' is such a group, n' is 1 or 2, and when n' is 2, the other R' group is different from said group.

In some embodiments, R' is a group of formula 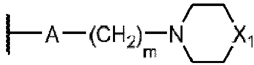 wherein A is O or NH, m is 2 and X₁ is O, CH₂ or N-CH₃, provided that when R' is such a group, n' is 1 or 2, and when n' is 2, the other R' group is different from said group.

In some embodiments, R' is a group of formula 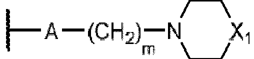 wherein A is O or NH, m is 3 and X₁ is O, CH₂ or N-CH₃, provided that when R' is such a group, n' is 1 or 2, and when n' is 2, the other R' group is different from said group.

In some embodiments, each R' is independently hydrogen, halogen, amino, methyl, -O-P(=O)-(OR₃)(OR₄), or a group of formula 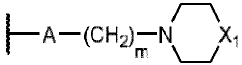 wherein A is O or NH, m is 2 or 3 and X₁ is O, CH₂ or N-CH₃, provided that when R' is such a group, n' is 1 or 2, and when n' is 2, the other R' group is different from said group.

In some embodiments, each R' is independently hydrogen, halogen, methyl, or a group of formula 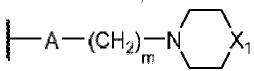 wherein A is O or NH, m is 2 and X₁ is O, CH₂ or N-CH₃, provided that when R' is such a group, n' is 1 or 2, and when n' is 2, the other R' group is different from said group.

In some embodiments, each R' is independently halogen, (C₁-C₃)alkyl, hydroxyl, -NR₁R₂, morpholinyl, morpholino, N-methylpiperazinyl, (C₁-C₃)fluoroalkyl, (C₁-C₄)alkoxy, or a group of formulas (**II**a) or (**IIIa**) as described herein.

In some embodiments, R' is halogen or methyl.

In some embodiments, each R' is independently selected from those depicted in **Tables 1-3,** below.

As described generally above, A is a covalent bond, oxygen, or NH.

In some embodiments, A is a covalent bond. In some embodiments, A is oxygen. In some embodiments, A is NH.

In some embodiments, A is selected from those depicted in **Tables 1-3,** below.

As described generally above, B is a covalent bond or NH.

In some embodiments, B is a covalent bond. In some embodiments, B is NH.

In some embodiments, B is selected from those depicted in **Tables 1-3,** below.

As described generally above, m is 1, 2, 3, 4 or 5.

In some embodiments, m is 1. In some embodiments, m is 2. In some embodiments, m is 3. In some embodiments, m is 4. In some embodiments, m is 5.

In some embodiments, m is selected from those depicted in **Tables 1-3,** below.

As described generally above, p is 1, 2 or 3.

In some embodiments, p is 1. In some embodiments, p is 2. In some embodiments, p is 3. In some embodiments, p is 4. In some embodiments, p is 5.

In some embodiments, p is selected from those depicted in **Tables 1-3,** below.

As described generally above, each of Ra and Rb is independently hydrogen, (C₁-C₅)alkyl, or (C₃-C₆)cycloalkyl, or Ra and Rb form together with the nitrogen atom to which they are attached a saturated 5- or 6-membered heterocycle, said heterocycle being optionally substituted by one or more Ra, provided that when R' is a group (IIa) or (IIIa), n' may be 2 or 3 only if other R' groups are different from said group (IIa) or (IIIa).

In some embodiments, Ra is hydrogen. In some embodiments, Ra is (C₁₋C₅)alkyl. In some embodiments, Ra is (C₃-C₆)cycloalkyl. In some embodiments, Rb is hydrogen. In some embodiments, Rb is (C₁-C₅)alkyl. In some embodiments, Rb is (C₃-C₆)cycloalkyl.

In some embodiments, Ra and Rb form together with the nitrogen atom to which they are attached a saturated 5- or 6- membered heterocycle, said heterocycle being optionally substituted by one or more Ra, provided that when R' is a group of formulas (IIa) or (IIIa), n' may be 2 or 3 only if other R' groups are different from said group of formulas (IIa) or (IIIa). In some embodiments, a saturated 5- or 6- membered heterocycle formed by Ra and Rb together with the nitrogen atom to which they are attached, as described above, optionally has an additional heteroatom selected from N, O and S.

In some embodiments, Ra and Rb form together with the nitrogen atom to which they are attached a saturated 5- or 6- membered heterocycle having an additional heteroatom selected from N, O and S, said heterocycle being substituted by one or more Ra, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 5-membered heterocycle, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 6-membered heterocycle, provided that when R' is a group of formulas (IIa) or (IIIa), n' may be 2 or 3 only if other R' groups are different from said group of formulas (IIa) or (IIIa).

In some embodiments, Ra and Rb form together with the nitrogen atom to which they are attached a saturated 5- or 6-membered heterocycle optionally having an additional heteroatom selected from N, O and S, said heterocycle being optionally substituted by one or more Ra, provided that when R' is a group (IIa) or (IIIa), n' may be 2 only if the other R' group is different from said group (IIa) or (IIIa).

In some embodiments, each of Ra and Rb is independently selected from those depicted in **Tables 1-3,** below.

As described generally above, R" is hydrogen, (C₁-C₄)alkyl, or a group of formula (IIa) as defined above.

In some embodiments, R" is hydrogen or (C₁-C₄)alkyl. In some embodiments, R" is hydrogen. In some embodiments, R" is (C₁-C₄)alkyl. In some embodiments, R" is a group of formula (IIa) as described herein.

In some embodiments, R" is a group of formula 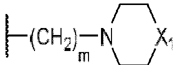 wherein m is 2 or 3, and X₁ is O, CH₂, or N-CH₃.

In some embodiments, R" is selected from those depicted in **Tables 1-3,** below.

In some embodiments, n is 1; n' is 1 or 2; R" is H; R is selected from methyl, methoxy, trifluoromethyl, halogen, trifluoromethoxy, and amino; and each R' is independently halogen, methyl, or a group 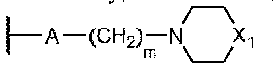 wherein A is O or NH, m is 2 or 3 and X₁ is O, CH₂ or N-CH₃, provided that when n' is 2, the other R' group is different from said group.

In some embodiments, n is 1; n' is 1; R" is H; R is selected from methyl, methoxy, trifluoromethyl, halogen, and trifluoromethoxy; and R' is halogen or methyl.

In some embodiments, the compound of formula (I) is a compound of formula (**Ia**): 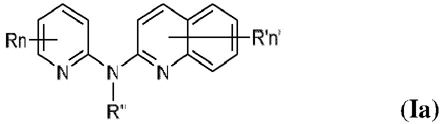 or a pharmaceutically acceptable salt thereof or prodrug thereof, wherein each of variables R, R', R", n, and n' is independently as defined above and described in embodiments

In some embodiments, the compound of formula (I) is a compound of formula (**Ib**): 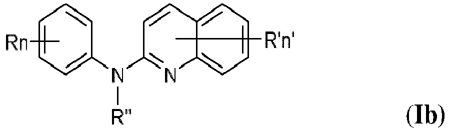 or a pharmaceutically acceptable salt thereof or prodrug thereof, wherein each of variables R, R', R", n, and n' is independently as defined above and described in embodiments herein, both singly and in combination.

In some embodiments, the present invention provides a combination, composition or kit wherein the compound of formula (I) is a compound of formula (Ib): 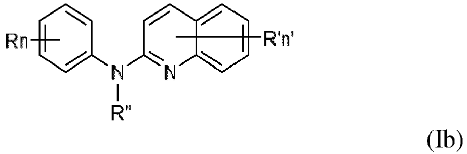 or anyone of its metabolites or a pharmaceutically acceptable salt thereof or prodrug thereof, wherein R, R' and R" are as defined above.

In some embodiments, the present invention provides a combination, composition or kit wherein the compound of formula (I) is a compound of formula (**Ic**): 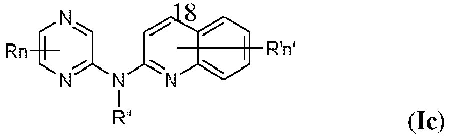 or a pharmaceutically acceptable salt thereof or prodrug thereof, wherein each of variables R, R', R", n, and n' is independently as defined above and described in embodiments herein, both singly and in combination.

In some embodiments, the present invention provides a combination, composition or kit wherein the compound of formula (I) is a compound of formula (**Id**): 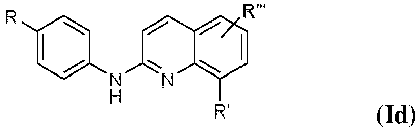 or a pharmaceutically acceptable salt thereof or prodrug thereof, wherein each of R and R' is independently as defined above and described in embodiments herein, both singly and in combination, and R'" is hydrogen or a group 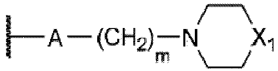 wherein A is O or NH, m is 2 or 3, and X₁ is O, CH₂ or N-CH₃.

In some embodiments, the present invention provides a combination, composition or kit wherein the compound of formula (I) is a compound of formula (**Id**), or a pharmaceutically acceptable salt thereof, wherein R is methyl, methoxy, trifluoromethyl, halogen, trifluoromethoxy, or amino; R' is halogen or methyl, and R'" is hydrogen or a group 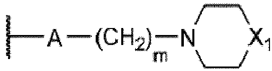 wherein A is O or NH, m is 2 or 3, and X₁ is O, CH₂ or N-CH₃.

In some embodiments, R'" is hydrogen.

In some embodiments, R'" is a group 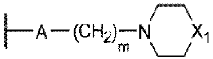 wherein A is O or NH, m is 2 or 3, and X₁ is O, CH₂ or N-CH₃. In some embodiments, R'" is a group 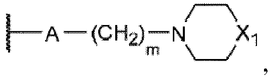 wherein A is O, m is 2 or 3, and X₁ is O, CH₂ or N-CH₃. In some embodiments, R'" is a group 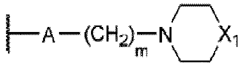 wherein A is NH, m is 2 or 3, and X₁ is O, CH₂ or N-CH₃.

In some embodiments, the present invention provides a combination, composition or kit wherein the compound of formula (I) is a compound of formula (**Ib'**): 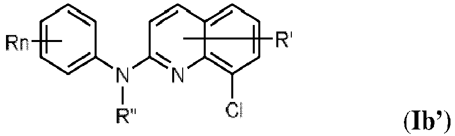 or a pharmaceutically acceptable salt thereof or prodrug thereof, wherein each of variables R, R', R", and n is independently as defined above and described in embodiments herein, both singly and in combination.

In some embodiments, the present invention provides a combination, composition or kit wherein the compound of formula (I) is a compound of formula (**Ib**), or a pharmaceutically acceptable salt thereof, wherein: each R is independently halogen, (C₁-C₃)fluoroalkyl, (C₁-C₃)fluoroalkoxy, -NR₁R₂, (C₁-C₄)alkoxy, or (C₁-C₃)alkyl, said alkyl being optionally mono- or di-substituted by a hydroxyl group; n is 1 or 2; n' is 1 or 2;
each of R₁ and R₂ is independently hydrogen or (C₁-C₃)alkyl;
each of R' is independently halogen, (C₁-C₃)alkyl, hydroxyl, -NR₁R₂, morpholinyl, morpholino, N-methylpiperazinyl, (C₁-C₃)fluoroalkyl, (C₁-C₄)alkoxy, or a group of formulas (**IIa**) or (**IIIa**) as described herein;
A is a covalent bond, oxygen, or NH; B is a covalent bond or NH;
m is 1, 2, 3, 4 or 5;
p is 1, 2 or 3;
each of Ra and Rb is independently hydrogen, (C₁-C₅)alkyl, or (C₃-C₆)cycloalkyl, or Ra and Rb form together with the nitrogen atom to which they are attached a saturated 5- or 6-membered heterocycle optionally having an additional heteroatom selected from N, O and S, said heterocycle being optionally substituted by one or more Ra, provided that when R' is a group (IIa) or (IIIa), n' may be 2 only if the other R' group is different from said group (IIa) or (IIIa); and R" is hydrogen or (C₁-C₄)alkyl.

In some embodiments, the present invention provides a combination, composition or kit wherein the compound of formula (I) is a compound of formula (**Ib**), or a pharmaceutically acceptable salt thereof, wherein each R' is independently hydrogen, halogen, (C₁-C₃)alkyl, or a (C₁-C₄)alkoxy group, said alkyl being optionally mono- or di-substituted by a hydroxyl group; R" is hydrogen or (C₁-C₄)alkyl; n is 1 or 2; n' is 1 or 2; when n is 1, R is (C₁-C₃) fluoroalkoxy, NR₁R₂, or phenoxy, wherein each of R₁ and R₂ is independently (C₁-C₃)alkyl; and when n is 2, one of the two R groups is (C₁-C₃) fluoroalkoxy and the other R group is (C₁-C₃)alkyl.

In some embodiments, the present invention provides a combination, composition or kit wherein the compound of formula (I) is a compound of formula (**Ib**), or a pharmaceutically acceptable salt thereof, wherein each R is independently (C₁-C₃)fluoroalkoxy; each R' is independently hydrogen, halogen, (C₁-C₃)alkyl, or (C₁-C₄)alkoxy; R" is hydrogen or (C₁-C₄)alkyl; n is 1; and n' is 1 or 2.

In some embodiments, the present invention provides a combination, composition or kit wherein the compound of formula (I) is a compound of formula (**Ib**'), or a pharmaceutically acceptable salt thereof, wherein each R is independently hydrogen, halogen, (C₁-C₃)alkyl, -NR₁R₂, (C₁-C₃)fluoroalkoxy, -NO₂, phenoxy, or (C₁-C₄)alkoxy, said alkyl being optionally mono- or di-substituted by a hydroxyl group; each of R₁ and R2 is independently hydrogen or (C₁-C₃)alkyl; R' is hydrogen, halogen, (C₁-C₃)alkyl, or (C₁-C₄)alkoxy, with the proviso that R' is different from a methyl group at position 4 of the quinoline group; R" is hydrogen or (C₁-C₄)alkyl; n is 1, 2, or 3; and n' is 1 or 2.

In some embodiments, the present invention provides a combination, composition or kit wherein the compound of formula (I) is a compound of formula (Ib') or anyone of its metabolites or a pharmaceutically acceptable salt thereof for use as defined above, wherein:
R independently represent a halogen atom or a group chosen among a (C₁-C₃)fluoroalkoxy group, a -NR₁R₂ group, a (C₁-C₄)alkoxy group, a -O-P(=O)(OR₃)(OR₄) group, a (C₁-C₃)alkyl group, a NO₂ group, a -A-(CH₂)ₘ-B-NRaRb group (formula IIa) and
a -(O-CH₂-CH₂)ₚ-O-Ra group (formula IIIa),
n is 1 or 2,
R' represents a hydrogen atom, a halogen atom or a group chosen among a -NR₁R₂ group, a -O-P(=O)(OR₃)(OR₄) group, a -NH-SO₂-N(CH₃)₂ group, and a -A-(CH₂)ₘ-B-NRaRb group (IIa),
R" is a hydrogen atom, a (C₁-C₄)alkyl group or a -A-(CH₂)ₘ-B-NRaRb group (formula IIa), R₁ and R₂ are independently a hydrogen atom or a (C₁-C₃)alkyl group,
R3 and R4 are independently hydrogen, Li⁺, Na⁺, K⁺, N⁺(Ra)₄ or benzyl,
A is a covalent bond, an oxygen atom or NH,
B is a covalent bond,
m is 2, 3 or 4,
p is 1, 2 or 3,

Ra and Rb independently represent a hydrogen atom or a (C₁-C₅)alkyl group, Ra and Rb can further form together with the nitrogen atom to which they are attached a saturated 5- or 6-membered heterocycle optionally containing a further heteroatom chosen among N, O and S, said heterocycle being optionally substituted by one or more Ra.

According to an even more particular embodiment, the invention provides a combination, composition or kit wherein the compound of formula (I) is a compound of formula (Ib') or anyone of its metabolites or a pharmaceutically acceptable salt thereof for use as defined above, wherein:
R independently represent F, Cl, -NH₂, -N(CH₃)₂, -OCH₃, -O-(CH₂)₃-CH₃, -OCF₃, -CH₃, -O-(CH₂)₂-OH, -O-(CH₂)₂-O-(CH₂)₂-OCH₃, a -NO₂ group, a -O-P(=O)(OH)(OH) group, a -O-(CH₂)₂-morpholino group or a -O-(CH₂)₂-piperidino group,
n is 1 or 2,
R' represents a hydrogen atom, Cl, -CH₂-CH₂-CH₃, a -O-(CH₂)₂-morpholino group, a -O-(CH₂)₂-piperidino group, a -O-(CH₂)₃-piperidino group, a -N-(CH₂)₃-morpholino group, a -NH-SO₂-N(CH₃)₂ group, NH₂, or a -O-P(=O)(OH)(OH) group, and R" is a hydrogen atom, -CH₃, a -(CH₂)₃-piperidino group, a -(CH₂)₂-morpholino group, a -(CH₂)₄-morpholino group or a -(CH₂)₂-pyrrolidino group.

In an embodiment, the present invention provides the compound of formula (Ib') or anyone of its metabolites or a pharmaceutically acceptable salt thereof for use as defined above, wherein said compound is chosen among: compounds 96, 98, 108, 109, 111, 115, 122, 125, 128, 129, 130, 132, 133, 135, 138 to 141, 143, and 145 to 164 as listed herein after.

8-Chloro-N-(4-(trifluoromethoxy)phenyl)quinolin-2-amine has the following formula: 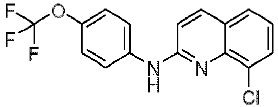 and is also called ABX464, which may in particular be implemented in the framework of the present invention.

In some embodiments, the compound ABX464, or a pharmaceutically acceptable salt thereof, is under an amorphous form. In some embodiments, the compound ABX464, or a pharmaceutically acceptable salt thereof, is under a crystallized form. In some embodiments, a crystallized form of the compound ABX464, or a pharmaceutically acceptable salt thereof, has a melting point at 120.5°C (± 2°C).

In some embodiments, a crystallized form of the compound ABX464, or a pharmaceutically acceptable salt thereof, shows peaks in an x-ray powder diffractogram (XRPD) at angles 7.3, 14.6, 18.4, and 24.9. In some embodiments, a crystallized form of the compound ABX464, or a pharmaceutically acceptable salt thereof, shows one or more XRPD peaks at angles selected from 18.0, 24.2, 28.3, and 29.5. In some embodiments, a crystallized form of the compound ABX464, or a pharmaceutically acceptable salt thereof, shows one or more XRPD peaks at angles selected from 18.6, 22.3, 23.0, and 23.5.

According to a particular embodiment, the crystalline polymorphic form of 8-Chloro-N-(4-(trifluoromethoxy)phenyl)quinolin-2-amine is characterized by the following main peaks expressed as degree 2-Theta angles by a XRPD analysis: 7.3, 14.6, 23.5, and 28.4 (each time ±0.2) and may further show the following additional peaks expressed as degree 2-Theta angles: 12.1, 17.3, 18.4, 23.0; 24.2, 24.9, 27.4 and 29.1 (each time ±0.2) and even optionally further the following additional peaks expressed as degree 2-Theta angles: 13.7, 16.3, 16.9, 18.1, 22.4, and 29.6 (each time ±0.2).

The compound of the invention may exist in the form of a free base or of pharmaceutically acceptable, namely of addition salts with pharmaceutically acceptable acids.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases.

Pharmaceutically acceptable salts of the compound of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

According to a particular embodiment, suitable physiologically acceptable acid addition salts of compound of the present invention include sulfate, hydrobromide, citrate, trifluoroacetate, ascorbate, hydrochloride, tartrate, triflate, maleate, mesylate, formate, acetate, fumarate and sulfonate, in particular alkylsufonate or arylsulfonate, and more particularly mesylate, triflate, edisylate, besylate and tosylate.

In some embodiments, the present invention provides a combination, composition or kit wherein the compound of formula (I) is a compound selected from **Table 1:**

**Table 1 (compounds of formula Ia as defined above)**

| | |
|---|---|
| **1** | 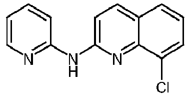 |
| **2** | 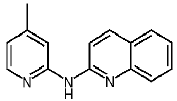 |
| **3** | 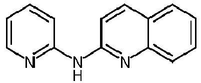 |
| **4** | 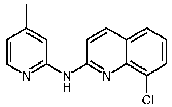 |
| **5** | 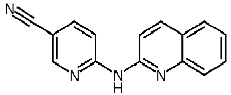 |
| **6** | 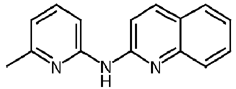 |
| **7** | 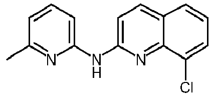 |
| **8** | 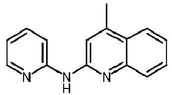 |
| **9** | 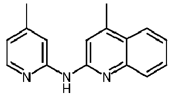 |
| **10** | 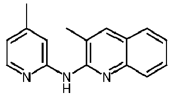 |
| **11** | 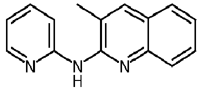 |
| **12** | 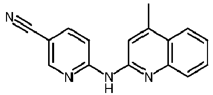 |
| **13** | 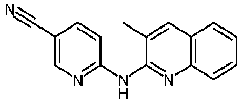 |
| **14** | 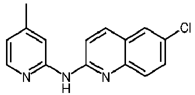 |
| **15** | 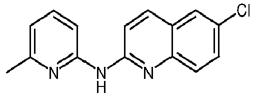 |
| **16** | 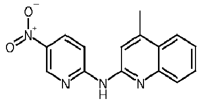 |
| **17** | 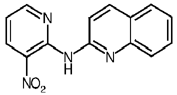 |
| **18** | 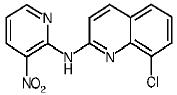 |
| **19** | 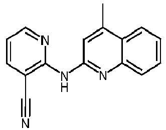 |
| **20** | 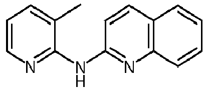 |
| **21** | 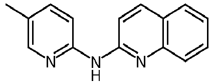 |
| **22** | 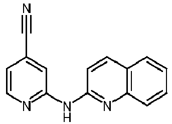 |
| **23** | 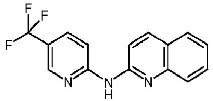 |
| **24** | 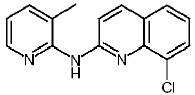 |
| **25** | 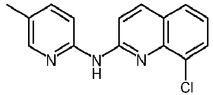 |
| **26** | 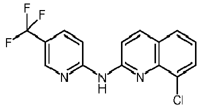 |
| **27** | 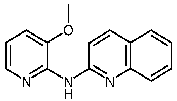 |
| **28** | 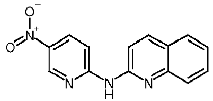 |
| **29** | 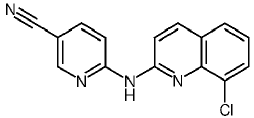 |
| **30** | 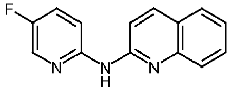 |
| **31** | 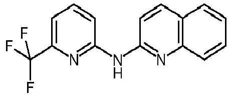 |
| **32** | 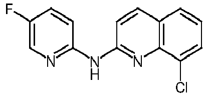 |
| **33** | 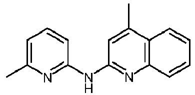 |
| **34** | 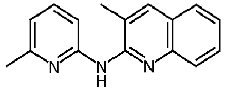 |
| **35** | 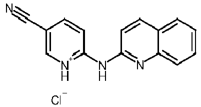 |
| **36** | 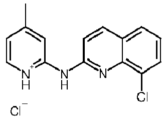 |
| **37** | 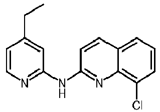 |
| **38** | 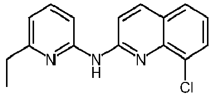 |
| **39** | 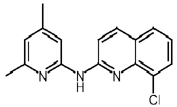 |
| **40** | 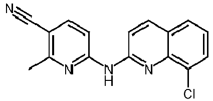 |
| **41** | 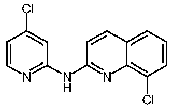 |
| **42** | 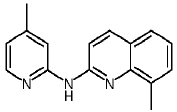 |
| **43** | 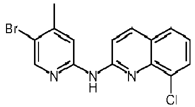 |
| **44** | 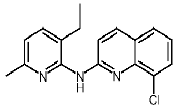 |
| **45** | 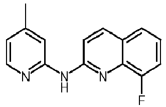 |
| **46** | 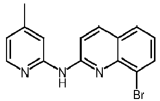 |
| **47** | 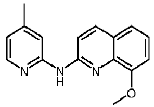 |
| **48** | 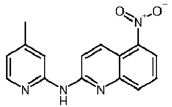 |
| **49** | 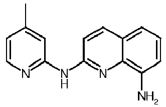 |
| **50** | 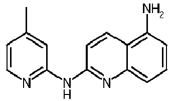 |
| **51** | 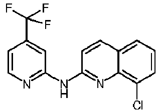 |
| **52** | 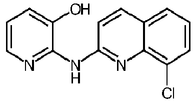 |
| **53** | 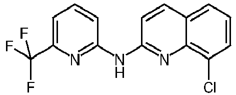 |
| **54** | 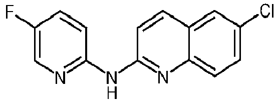 |
| **55** | 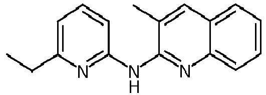 |
| **56** | 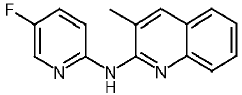 |
| **57** | 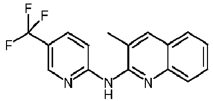 |
| **58** | 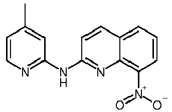 |
| **59** | 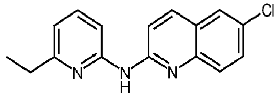 |
| **60** | 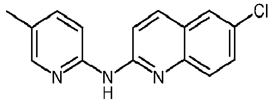 |
| **61** | 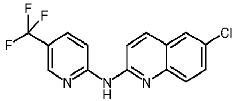 |
| **62** | 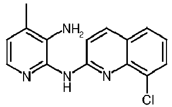 |
| **63** | 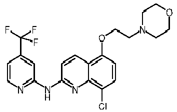 |
| **64** | 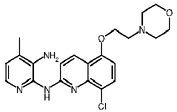 |
| **65** | 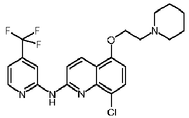 |
| **66** | 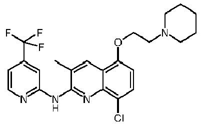 |
| **67** | 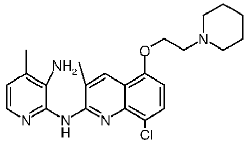 |
| **68** | 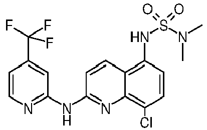 |
| **69** | 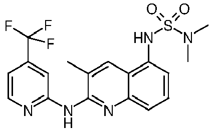 |
| **70** | 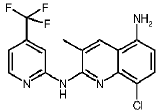 |
| **71** | 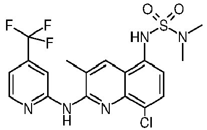 |
| **72** | 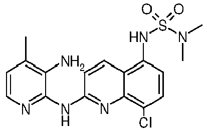 |
| **73** | 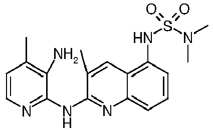 |
| **74** | 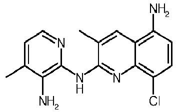 |
| **75** | 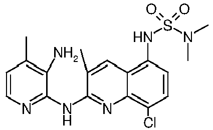 |
| **76** | 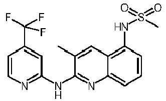 |
| **77** | 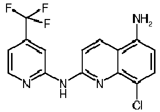 |
| **78** | 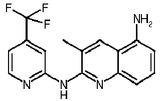 |
| **79** | 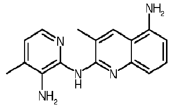 |
| **80** | 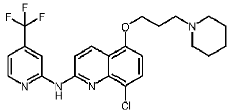 |
| **81** | 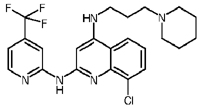 |
| **82** | 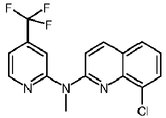 |
| **83** | 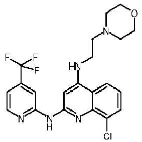 |
| **84** | 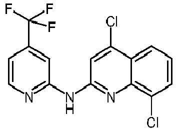 |
| **85** | 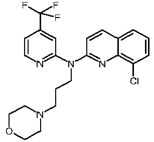 |
| **86** | 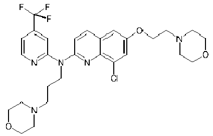 |
| **87** | 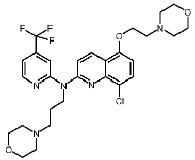 |
| **88** | 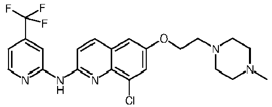 |
| **89** | 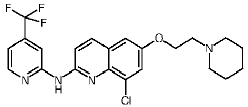 |
| **90** | 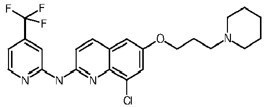 |
| **91** | 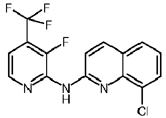 |
| **92** | 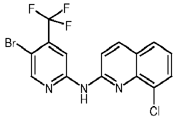 |
| **93** | 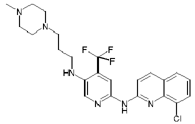 |
| **94** | 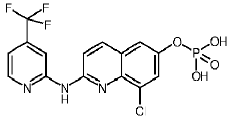 |

or a pharmaceutically acceptable salt thereof or a prodrug thereof.

In some embodiments, the present invention provides a combination, composition or kit wherein the compound of formula (I) is a compound selected from **Table 2:**

**Table 2 (compounds of formula (Ib) as defined above)**

| | |
|---|---|
| **95** | 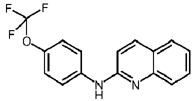 |
| **96** | 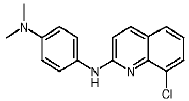 |
| **97** | 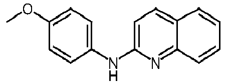 |
| **98** | 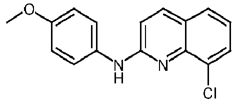 |
| **99** | 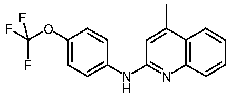 |
| **100** | 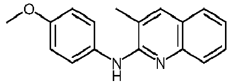 |
| **101** | 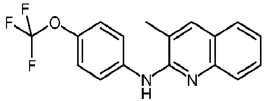 |
| **102** | 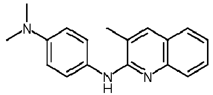 |
| **103** | 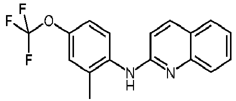 |
| **104** | 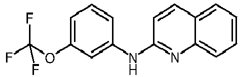 |
| **105** | 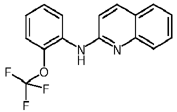 |
| **106** | 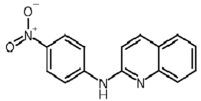 |
| **107** | 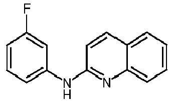 |
| **108** | 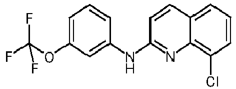 |
| **109** | 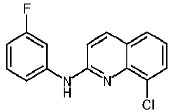 |
| **110** | 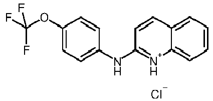 |
| **111** | 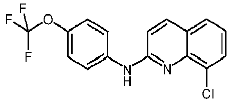 |
| **112** | 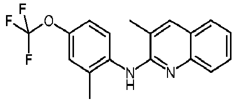 |
| **113** | 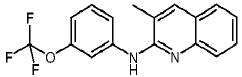 |
| **114** | 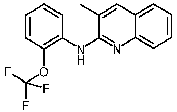 |
| **115** | 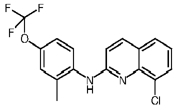 |
| **116** | 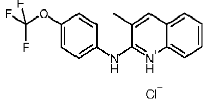 |
| **117** | 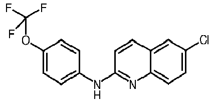 |
| **118** | 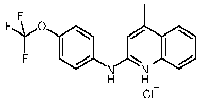 |
| **119** | 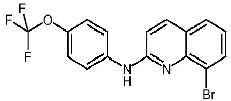 |
| **120** | 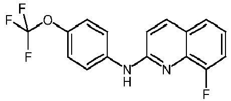 |
| **121** | 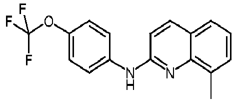 |
| **122** | 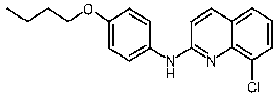 |
| **123** | 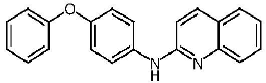 |
| **124** | 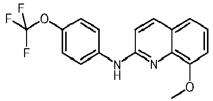 |
| **125** | 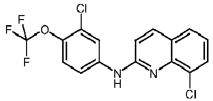 |
| **126** | 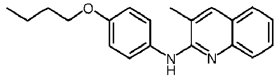 |
| **127** | 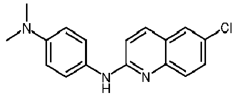 |
| **128** | 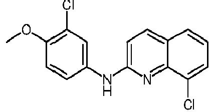 |
| **129** | 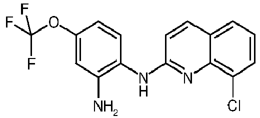 |
| **130** | 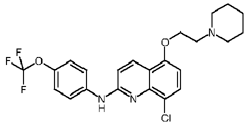 |
| **131** | 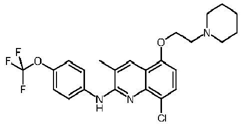 |
| **132** | 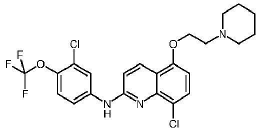 |
| **133** | 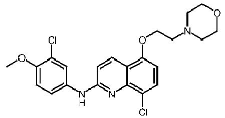 |
| **134** | 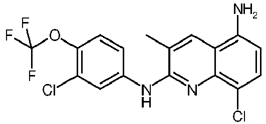 |
| **135** | 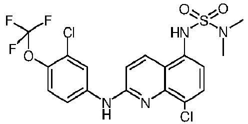 |
| **136** | 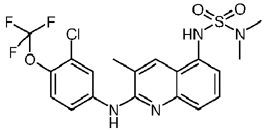 |
| **137** | 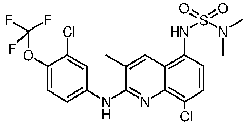 |
| **138** | 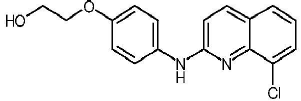 |
| **139** | 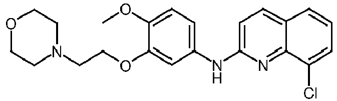 |
| **140** | 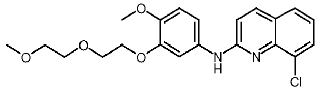 |
| **141** | 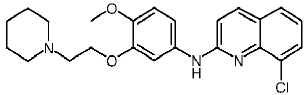 |
| **142** | 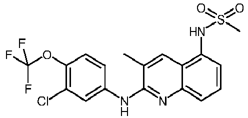 |
| **143** | 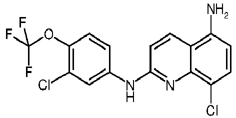 |
| **144** | 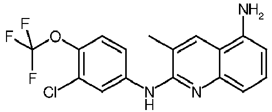 |
| **145** | 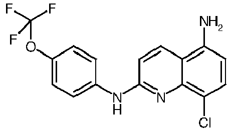 |
| **146** | 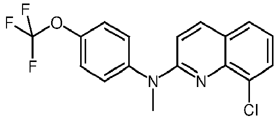 |
| **147** | 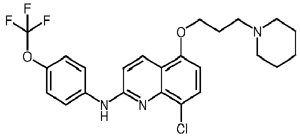 |
| **148** | 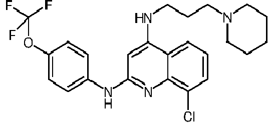 |
| **149** | 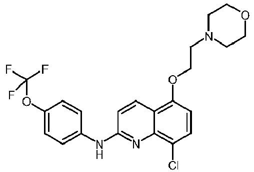 |
| **150** | 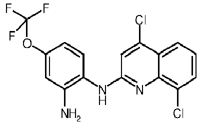 |
| **151** | 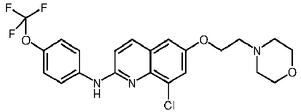 |
| **152** | 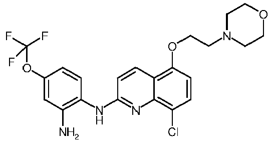 |
| **153** | 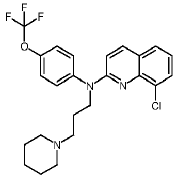 |
| **154** | 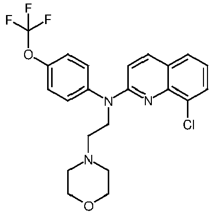 |
| **155** | 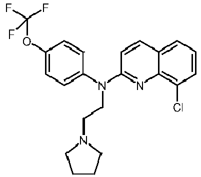 |
| **156** | 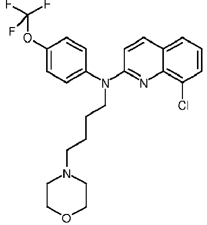 |
| **157** | 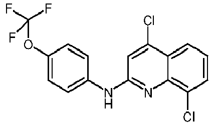 |
| **158** | 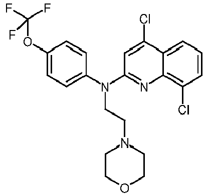 |
| **159** | 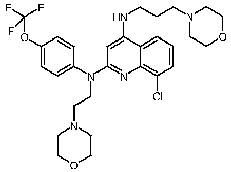 |
| **160** | 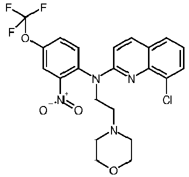 |
| **161** | 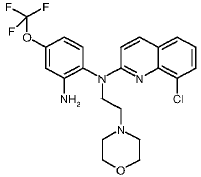 |
| **162** | 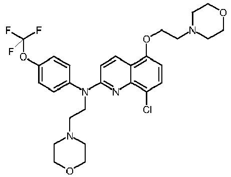 |
| **163** | 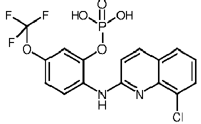 |
| **164** | 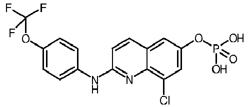 |

or a pharmaceutically acceptable salt thereof or a prodrug thereof.

In some embodiments, the present invention provides a combination, composition or kit wherein the compound of formula (I) is a compound selected from **Table 3:**

**Table 3 (compounds of formula (I) other that compounds (Ia) and (Ib))**

| | |
|---|---|
| **165** | 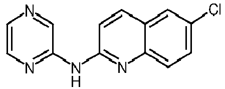 |
| **166** | 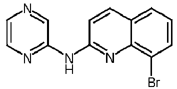 |
| **167** | 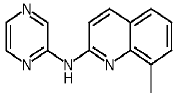 |
| **168** | 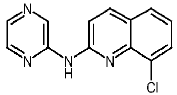 |
| **169** | 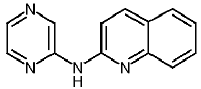 |
| **170** | 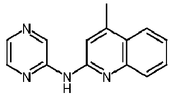 |
| **171** | 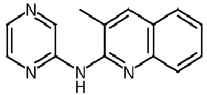 |
| **172** | 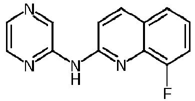 |
| **173** | 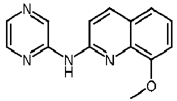 |
| **174** | 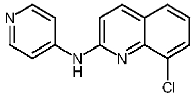 |
| **175** | 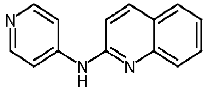 |
| **176** | 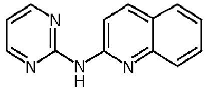 |
| **177** | 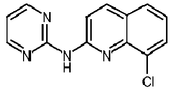 |
| **178** | 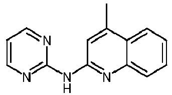 |
| **179** | 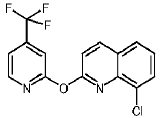 |
| **180** | 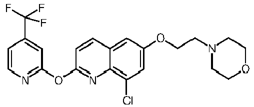 |
| **181** | 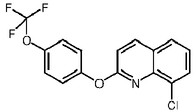 |
| **182** | 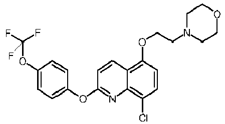 |
| **183** | 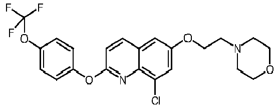 |

or a pharmaceutically acceptable salt thereof or a prodrug thereof.

In some embodiments, a compound described herein is in a salt form selected from sulfate, hydrobromide, citrate, trifluoroacetate, ascorbate, hydrochloride, tartrate, triflate, maleate, mesylate, formate, acetate, fumarate, and sulfonate. In some embodiments, a compound described herein is in salt form as alkylsufonate or arylsulfonate. In some embodiments, a compound described herein is in salt form as mesylate, triflate, edisylate, besylate and tosylate.

In one aspect, the present invention provides a combination, composition or kit wherein the compound of formula (I) or metabolite thereof is under the form of a metabolite thereof. In some embodiments, the present invention provides a combination, composition or kit wherein the compound of formula (I) is under the form of a N-glucuronide metabolite of compound of formula (I) as described herein.

In some embodiments, the present invention provides a combination, composition or kit wherein the compound of formula (I) or metabolite thereof is under the form of a compound of formula (**IV**): 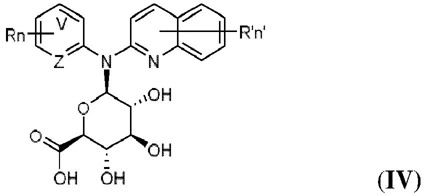 or a pharmaceutically acceptable salt thereof, wherein each of variables V, Z, R, R', n, and n' is as defined above and described in embodiments herein, both singly or in combination.

In some embodiments, the present invention provides a combination, composition or kit wherein the compound of formula (I) or metabolite thereof is under the form of a compound of formula (**IVa**): 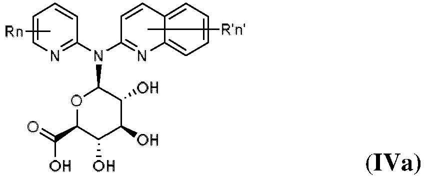 or a pharmaceutically acceptable salt thereof, wherein each of variables R, R', n, and n' is independently as defined above and described in embodiments herein, both singly and in combination.

In some embodiments, the present invention provides a combination, composition or kit wherein the compound of formula (I) or metabolite thereof is under the form of a compound of formula (**IVb**): 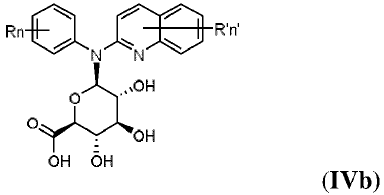 or a pharmaceutically acceptable salt thereof, wherein each of variables R, R', n, and n' is independently as defined above and described in embodiments herein, both singly and in combination.

In some embodiments, the present invention provides a combination, composition or kit wherein the compound of formula (I) or its metabolite is under ther form of a compound of formula (**IVc**): 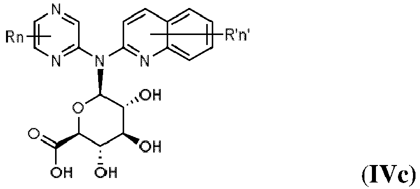 or a pharmaceutically acceptable salt thereof, wherein each of variables R, R', n, and n' is independently as defined above and described in embodiments herein, both singly and in combination.

In some embodiments, the present invention provides a combination, composition or kit wherein the compound of formula (I) or its metabolite is under the form of a compound of formula (**IVb'**): 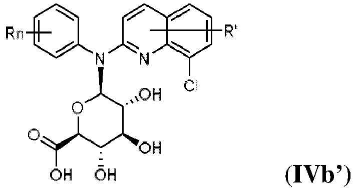 or a pharmaceutically acceptable salt thereof, wherein each of variables R, R', and n is independently as defined above and described in embodiments herein, both singly and in combination.

In some embodiments, the present invention provides a combination, composition or kit wherein the compound of formula (I) or its metabolite is under the form of a compound of formula (**IVd**): 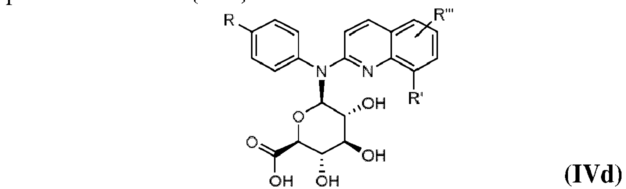 or a pharmaceutically acceptable salt thereof, wherein each of variables R, R', R'" is independently as defined above and described in embodiments herein, both singly and in combination.

According to one embodiment, the combination, composition or kit of the invention comprises a 8-Chloro-N-(4-(trifluoromethoxy)phenyl)quinolin-2-amine metabolite and a S1P receptor modulator or a pharmaceutically acceptable salt thereof. Said metabolite may be a glucuronide metabolite, having the following formula:

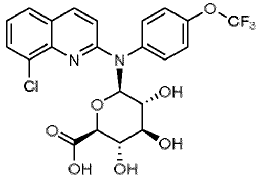

In some embodiments, the metabolite of compound of formula (I) or salt thereof, as for the 8-Chloro-N-(4-(trifluoromethoxy)phenyl)quinolin-2-amine glucuronide metabolite as recited above is under amorphous form.

### S1P receptor modulator

The S1P receptor modulator is also called an antagonist of the sphingosine-1-phosphate-1 (S1P) receptor.

According to a particular embodiment, the S1P receptor modulator is selected from ponesimod, cenerimod, siponimod, fingolimod, ozanimod and etrasimod, and pharmaceutically acceptable salts thereof.

According to a particular embodiment, the S1P receptor modulator is Ponesimod, also called (R)-5-[3-chloro-4-(2,3-dihydroxy-propoxy)-benz[Z]ylidene]-2-([Z]-propylimino)-3-o-tolyl-thiazolidin- 4-one, CAS Reg. No. 854107-55-4, as described in WO2005/054215, WO2008/062376, WO2010/046835, WO2014/027330, which is a selective S1P1 receptor agonist and oral administration thereof results in a consistent, sustained, and dose-dependent reduction in the number of peripheral blood lymphocytes. Ponesimod has been described to be useful in the treatment and/or prevention of diseases or disorders associated with an activated immune system (see e.g. WO2005/054215 and WO2009/115954). In particular ponesimod has shown clinical benefit in phase II / phase III trials in patients with moderate to severe chronic plaque psoriasis and in patients with relapsing-remitting multiple sclerosis.

According to a particular embodiment, the S1P receptor modulator is Cenerimod, also called (S)-3-[[4-[5-(2-Cyclopentyl-6-methoxypyridin-4-yl)[1,2,4]oxadiazol-3-yl]-2-ethyl-6-methylphenyl]-oxy]-propane-1,2-diol, CAS Reg. No. 1262414-04-9, as described in WO2011/007324, WO2013/175397, WO2016/184939, Piali et al., Pharmacol Res Perspect. 2017;e00370, which is a selective S1P1 receptor agonist and entered a multiple-dose efficacy and safety study for the treatment of systemic lupus erythematosus.

According to a particular embodiment, the S1P receptor modulator is Siponimod, also called 1-(4-[1-[(E)-4-Cyclohexyl-3-trifluoromethyl-benzyloxyiminol-ethyl]-2-ethyl-benzyl)-azetidine-3- carboxylic acid, CAS Reg. No. 1230487-00-9, as described in WO2004/103306, WO2010/071794, WO2010/080409, WO2010/080455, WO2019/064184, which is an S1P receptor modulator and was studied for the treatment of secondary progressive multiple sclerosis (SPMS), which is the progressive neurological decline of multiple sclerosis that happens independent of acute relapses. In active SPMS, siponimod decreases the risk of disability and MS relapses. In the U.S., siponimod is indicated for the treatment of relapsing forms of multiple sclerosis (MS), to include clinically isolated syndrome (defined as a first episode of neurologic symptoms that lasts at least 24 h and is caused by inflammation or demyelination in the central nervous system), relapsing-remitting disease, and active secondary progressive disease (SPMS), in adults with a recommended maintenance dosage of 2 mg orally once-daily.

According to a particular embodiment, the S1P receptor modulator is Fingolimod, also called 2-amino-2-[2-(4-octylphenyl)ethyl]-propane-1,3-diol, CAS Reg. No. 162359-55-9, as described in WO2008/000419, WO2010/055027, WO2010/055028, WO2010/072703, which is a non-selective S1P receptor modulator indicated for the treatment of the relapsing form of multiple sclerosis (MS).

According to a particular embodiment, the S1P receptor modulator is Ozanimod, also called 5-[3-[(S)-2,3-Dihydro-1-[(2-hydroxyethyl)amino]-1H-inden-4-yl]-1,2,4-oxadiazol-5-yl]-2-(1-methylethoxyj-benzonitrile, CAS Reg. No. 1306760-87-1, as described in WO2011/060392, WO2015/066515, WO2018/184185. WO2018/208855, WO2018/215807, WO2019/058290, WO2019/094409, which is an investigational S1P receptor modulator that was tested in phase III clinical trials for the therapy of relapsing forms of multiple sclerosis (RMS); and is further tested in Crohn's disease and ulcerative colitis (UC). Since 2020, ozanimod is indicated in the U.S. for the treatment of relapsing forms of multiple sclerosis (MS), to include clinically isolated syndrome, relapsing-remitting disease, and active secondary progressive disease, in adults; and in Europe for the treatment of adult patients with relapsing remitting multiple sclerosis (RRMS) with active disease as defined by clinical or imaging features.

According to a particular embodiment, the S1P receptor modulator is Etrasimod, which is an oral, once-daily, selective S1P receptor modulator designed to reduce lymphocyte levels.

Etrasimod or APD334, also called [(3R)-7-[[4-Cyclopentyl-3-(trifluoromethyl)phenyl]methoxy]-1,2,3,4-tetrahydrocyclopent[b]indole-3- acetic acid, CAS Reg. No. 1206123-37-6, or a pharmaceutically acceptable salt, solvate, or hydrate thereof, is an investigational drug candidate intended for the treatment of sphingosine 1-phosphate subtype 1 (S1P) receptor-associated disorders.

In particular said compound is an investigational S1P receptor modulator that is currently in development for example for the therapy of inflammatory bowel diseases including Crohn's disease, ulcerative colitis (UC) as well as eosinophilic esophagitis:

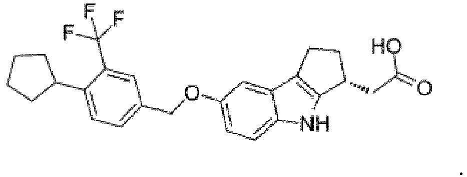

Investigations on dermatological indications are also ongoing, and namely for atopic dermatitis and for alopecia areata.

Said compound has for example been described in WO2010/011316. Processes for manufacturing and crystalline forms of Compound 1, or a pharmaceutically acceptable salt, solvate, or hydrate thereof, are also disclosed within WO2011094008.

Further S1P1 receptor modulators may be cited, that may also be encompassed within the framework of the present invention, and namely Amiselimod (MT-1303, 2-Amino-2-[2-[4-(heptyloxy)-3-(trifluoromethyl)phenyl]ethyl]-1,3-propanediol, CAS Reg. No. 942399-20-4); Ceralifimod (1 -[[3,4-dihydro-6-[(2-methoxy-4-propylphenyl)methoxy]-1 -methyl-2-naphthalenyl] methyl]-3-azetidinecarboxylic acid, CAS Reg. No. 891859-12-4); GSK 2018682 (4-[5-[5-Chloro-6-(1-methylethoxy)-3-pyridinyl]-1,2,4-oxadiazol-3-yl]-1 H-indole-1 - butanoic acid, as described in WO2008/074821); CS-0777 (1-[5-[(3R)-3-Amino-4-hydroxy-3-methylbutyl]-1-methyl-1 H-pyrrol-2-yl]-4-(4-methylphenyl)- 1-butanone, CAS Reg. No. 827344-05-8); and Mocravimod (2-amino-2-[2-(2-chloro-4-{ [3-(phenylmethoxy)phenyl]sulfanyl}phenyl)ethyl]propane-1,3- diol; KEP203, CAS Reg. No. 509092-16-4), disclosed to enter studies in high-risk acute myeloid leukemia.

### Pharmaceutical indications

The pharmaceutical composition according to the present invention may be used in the treatment of a variety of inflammatory diseases, disorders of conditions.

An inflammatory disease can for example be selected in the list consisting of: an inflammatory disease associated with an autoimmune disease, a central nervous system (CNS) inflammatory disease, a joint inflammation disease, an inflammatory digestive tract disease, inflammatory skin and other inflammatory diseases related to epithelial cells, inflammation associated with cancer, inflammation associated with irritation, and inflammation associated with injury.

According to one embodiment, the inflammatory disease, disorder or condition is selected from:
(a) an inflammatory disease, disorder, or condition in the pancreas selected from diabetes type-1, diabetes type-2, acute and chronic pancreatitis;
(b) an inflammatory disease, disorder, or condition in the kidney selected from glomerulosclerosis, glomerulonephritis, nephritis, acute kidney injury, Berger's disease, Goodpasture's syndrome, Wegener's granulomatosis and kidney transplant acute or chronic rejection;
(c) an inflammatory disease, disorder, or condition in the liver selected from nonalcoholic steatohepatitis (NASH), non-alcoholic fatty liver disease (NAFLD), cholestatic liver disease, sclerosing cholangitis and liver transplant acute or chronic rejection;
(d) an inflammatory disease, disorder, or condition in the lung or heart selected from bronchitis, asthma, chronic obstructive pulmonary disease (COPD), pulmonary fibrosis, pulmonary hypertension, sarcoidosis, myocarditis, pericarditis and lung or heart transplant acute or chronic rejection, *Coronaviridae* infection and conditions related thereto, in particular wherein the *Coronaviridae* is a Sarbecovirus selected from Severe Acute Respiratory Syndrome-related coronaviruses, even more particularly wherein the Severe Acute Respiratory Syndrome (SARS)-related coronaviruses are selected from the group consisting of: SARS-CoV, SARSr-CoV WIV1, SARSr-CoV HKU3, SARSr-CoV RP3, SARS-CoV-2; including including strains responsible for COVID-19 and their mutants;
(e) an inflammatory disease, disorder, or condition in the skin selected from psoriasis, dermatitis, such as eczema, contact dermatitits, atopic dermatitis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acnea, keloid scar, and other inflammatory or allergic conditions of the skin;
(e) an inflammatory disease, disorder, or condition in the vessel/blood selected from Behcet's disease, vasculitis, sepsis, tumor angiogenesis, proliferative vascular disease and restenosis, atherosclerosis;
(f) an inflammatory disease, disorder, or condition in the eye selected from conjunctivitis, scleritis, episcleritis, panuveitis, choroiditis, chorioretinitis, neuroretinitis, uveitis, orbital inflammatory disease, and optical neuritis;
(g) an inflammatory disease, disorder, or condition in the central or peripheral nervous system selected from non-viral and viral encephalitis and meningitis, depression, neuropathic pain, including chronic pain, traumatic brain injury, including stroke, neurodegenerative diseases such as Alzheimer's disease, and Parkinson disease, Myelitis, Charcot-Marie-Tooth disease type 1 (including CMT1A and CMT1B), Amyotrophic lateral sclerosis (ALS), Creutzfeldt-Jakob disease, demyelinating polyneuropathy and peripheral neuropathy;
(i) an autoimmune disease, disorder, or condition selected from Sjogren's syndrome, Lupus, including in the skin and kidney, Guillain-Barre syndrome, Myasthenia gravis, Hashimoto's thyroiditis, idiopathic purpura, aplastic anemia, Graves disease, and Myocarditis;
(k) an inflammatory disease, disorder, or condition in the reproductive system selected from endometriosis, uterine fibroma, prostate dysplasia or growth, and cervix dysplasia;
(l) an inflammatory disease, disorder, or condition in the bone and/or joints selected from Rheumatoid arthritis (RA), osteoarthritis (OA); ankylosing spondylitis, juvenile idiopathic arthritis, psoriatic arthritis, periodontitis, and hand, foot, ankle, knee, hip, shoulder, elbow or spine arthritis and/or demineralization;
(m) an inflammatory disease, disorder, or condition in the digestive tract selected from inflammation associated with colon carcinoma, Inflammatory Bowel Disease, including Crohn's disease, Ulcerative Colitis and eosinophilic esophagitis; and
(n) an inflammatory disease, disorder, or condition in the central nervous system selected from Multiple sclerosis (MS), relapsing-remitting multiple sclerosis (RRMS); relapsing forms of multiple sclerosis (RMS) and secondary progressive multiple sclerosis (SPMS).

### Inflammatory Diseases, Disorders or Conditions

Combinations as described herein are useful in the treatment of inflammatory or obstructive airways diseases, resulting, for example, in reduction of tissue damage, airways inflammation, bronchial hyperreactivity, remodeling or disease progression. In some embodiments, an inflammatory disease, disorder, or condition is inflammatory or obstructive airways diseases including, but not limited to, asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics.

Combinationss as described herein are useful in the treatment of heteroimmune diseases. In some embodiments, an inflammatory disease, disorder, or condition is heteroimmune diseases including, but not limited to, graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis.

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, such as therapy for or intended to restrict or abort symptomatic attack when it occurs, for example anti-inflammatory or bronchodilatory. Prophylactic benefit in asthma may in particular be apparent in subjects prone to "morning dipping". "Morning dipping" is a recognized asthmatic syndrome, common to a substantial percentage of asthmatics and characterized by asthma attack, e.g. between the hours of about 4 to 6 am, i.e. at a time normally substantially distant form any previously administered symptomatic asthma therapy.

In some embodiments, an inflammatory disease, disorder, or condition is selected from acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. In some embodiments, an inflammatory disease, disorder, or condition is bronchitis, wherein the bronchitis is of whatever type or genesis including, but not limited to, acute, arachidic, catarrhal, croupus, chronic or phthinoid bronchitis. In some embodiments, an inflammatory disease, disorder, or condition is pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

In some embodiments, an inflammatory disease, disorder, or condition is an eosinophil related disorder, e.g. eosinophilia. In some embodiments, an eosinophil related disorder is an eosinophil related disorder of the airways (e.g. involving morbid eosinophilic infiltration of pulmonary tissues) including hypereosinophilia as it effects the airways and/or lungs as well as, for example, eosinophil-related disorders of the airways consequential or concomitant to Loffler's syndrome, eosinophilic pneumonia, parasitic (in particular metazoan) infestation (including tropical eosinophilia), bronchopulmonary aspergillosis, polyarteritis nodosa (including Churg- Strauss syndrome), eosinophilic granuloma and eosinophil-related disorders affecting the airways occasioned by drug-reaction.

Combinations as described herein are also useful in the treatment of inflammatory or allergic conditions of the skin. In some embodiments, an inflammatory or allergic condition of the skin is selected from psoriasis, dermatitis such as eczema, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, systemic lupus erythematosus, pemphigus vulgaris, pemphigus foliaceus, paraneoplastic pemphigus, epidermolysis bullosa acquisita, acne vulgaris, and other inflammatory or allergic conditions of the skin.

In some embodiments, an inflammatory disease, disorder, or condition is a disease or condition having an inflammatory component, for example, diseases and conditions of the eye such as ocular allergy, conjunctivitis, keratoconjunctivitis sicca, uveitis and vernal conjunctivitis, diseases and conditions affecting the nose including allergic rhinitis, and inflammatory disease in which autoimmune reactions are implicated or having an autoimmune component or etiology, including autoimmune hematological disorders (e.g. hemolytic anemia, aplastic anemia, pure red cell anemia and idiopathic thrombocytopenia), systemic lupus erythematosus, rheumatoid arthritis, polychondritis, scleroderma, Wegener granulamatosis, dermatomyositis, chronic hepatitis, myasthenia gravis, Steven-Johnson syndrome, idiopathic sprue, autoimmune inflammatory bowel disease (e.g. ulcerative colitis and Crohn's disease), irritable bowel syndrome, celiac disease, periodontitis, hyaline membrane disease, kidney disease, glomerular disease, alcoholic liver disease, multiple sclerosis, endocrine opthalmopathy, Grave's disease, sarcoidosis, alveolitis, chronic hypersensitivity pneumonitis, multiple sclerosis, primary biliary cirrhosis, uveitis (anterior and posterior), Sjogren's syndrome, keratoconjunctivitis sicca, uveitis, and vernal keratoconjunctivitis, interstitial lung fibrosis, psoriatic arthritis, systemic juvenile idiopathic arthritis, cryopyrin-associated periodic syndrome, Muckle-Wells syndrome, nephritis, vasculitis, diverticulitis, interstitial cystitis, glomerulonephritis (with and without nephrotic syndrome, e.g. including idiopathic nephrotic syndrome or minal change nephropathy), chronic granulomatous disease, endometriosis, leptospiriosis renal disease, glaucoma, retinal disease, ageing, headache, pain, complex regional pain syndrome, cardiac hypertrophy, musclewasting, catabolic disorders, obesity, fetal growth retardation, intestinal failure, hyperchlolesterolemia, heart disease, chronic heart failure, mesothelioma, anhidrotic ecodermal dysplasia, Behcet's disease, incontinentia pigmenti, Paget's disease, acute or chronic pancreatitis, hereditary periodic fever syndrome, asthma (allergic and non-allergic, mild, moderate, severe, bronchitic, and exercise-induced), acute lung injury, acute respiratory distress syndrome, eosinophilia, hypersensitivities, anaphylaxis, nasal sinusitis, ocular allergy, silica induced diseases, COPD (reduction of damage, airways inflammation, bronchial hyperreactivity, remodeling or disease progression), pulmonary disease, cystic fibrosis, acid-induced lung injury, pulmonary hypertension, polyneuropathy, cataracts, muscle inflammation in conjunction with systemic sclerosis, inclusion body myositis, myasthenia gravis, thyroiditis, Addison's disease, lichen planus, Type 1 diabetes, or Type 2 diabetes, appendicitis, atopic dermatitis, asthma, allergy, blepharitis, bronchiolitis, bronchitis, bursitis, cervicitis, cholangitis, cholecystitis, chronic graft rejection, colitis, conjunctivitis, Crohn's disease, cystitis, dacryoadenitis, dermatitis such as asthma, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, or vulvitis.

In some embodiments, an inflammatory disease, disorder, or condition is acute or chronic graft rejection in kidney, liver, heart, pulmonary transplantation, or graft versus-host disease in bone marrow graft.

In some embodiments, an inflammatory disease, disorder, or condition is an inflammatory disease, disorder, or condition of the skin. In some embodiments, an inflammatory disease, disorder, or condition of the skin is selected from contact dermatitits, atopic dermatitis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, and other inflammatory or allergic conditions of the skin.

In some embodiments, an inflammatory disease, disorder, or condition is selected from acute and chronic gout, chronic gouty arthritis, psoriasis, psoriatic arthritis, rheumatoid arthritis, Juvenile rheumatoid arthritis, Systemic jubenile idiopathic arthritis (SJIA), Cryopyrin Associated Periodic Syndrome (CAPS), Muckle-Wells syndrome, and osteoarthritis.

In some embodiments, an inflammatory disease, disorder, or condition is a TH17 mediated disease. In some embodiments, a TH17 mediated disease is selected from Systemic lupus erythematosus, Multiple sclerosis, and inflammatory bowel disease (including Crohn's disease or ulcerative colitis).

In some embodiments, an inflammatory disease, disorder, or condition is selected from Sjogren's syndrome, allergic disorders, osteoarthritis, conditions of the eye such as ocular allergy, conjunctivitis, keratoconjunctivitis sicca and vernal conjunctivitis, and diseases affecting the nose such as allergic rhinitis.

In some embodiments, an inflammatory disease, disorder, or condition is associated with transplantation. In some embodiments, an inflammatory disease, disorder, or condition is associated with organ transplantation, organ transplant rejection, and/or graft versus host disease. In some embodiments, an inflammatory disease, disorder, or condition is an autoimmune disorder. In some embodiments an autoimmune disorder is type 1 diabetes, systemic lupus erythematosus, multiple sclerosis, psoriasis, Behçet's disease, POEMS syndrome, Crohn's disease, ulcerative colitis, ankylosing spondylitis, axial spondyloarthritis, primary biliary cirrhosis, autoimmune hepatitis, or inflammatory bowel disease.

In some embodiments, an inflammatory disease, disorder, or condition is an inflammatory disorder. In some embodiments, an inflammatory disorder is rheumatoid arthritis, asthma, chronic obstructive pulmonary disease, psoriasis, hepatomegaly, Crohn's disease, ulcerative colitis, ankylosing spondylitis, axial spondyloarthritis, primary biliary cirrhosis, polymyalgia rheumatica, giant cell arteritis, or inflammatory bowel disease.

In some embodiments, the present invention provides a method for treating an inflammatory disease, disorder, or condition in the pancreas. In some embodiments, an inflammatory disease, disorder, or condition in the pancreas is selected from diabetes type-1, diabetes type-2, acute and chronic pancreatitis.

In some embodiments, the present invention provides a method for treating an inflammatory disease, disorder, or condition in the kidney. In some embodiments, an inflammatory disease, disorder, or condition in the kidney is selected from glomerulosclerosis, glomerulonephritis, nephritis, acute kidney injury, Berger's disease, Goodpasture's syndrome, Wegener's granulomatosis, and kidney transplant acute or chronic rejection.

In some embodiments, the present invention provides a method for treating an inflammatory disease, disorder, or condition in the liver. In some embodiments, an inflammatory disease, disorder, or condition in the liver is selected from nonalcoholic steatohepatitis (NASH), non-alcoholic fatty liver disease (NAFLD), cholestatic liver disease, sclerosing cholangitis, and liver transplant acute or chronic rejection.

In some embodiments, the present invention provides a method for treating an inflammatory disease, disorder, or condition in the lung or heart. In some embodiments, an inflammatory disease, disorder, or condition in the lung is selected from chronic obstructive pulmonary disease (COPD), asthma, pulmonary fibrosis, pulmonary hypertension, sarcoidosis, myocarditis, pericarditis and lung or heart transplant acute or chronic rejection.

In some embodiments, the present invention provides a method for treating an inflammatory disease, disorder, or condition in the skin. In some embodiments, an inflammatory disease, disorder, or condition in the skin is selected from contact dermatitits, atopic dermatitis, psoriasis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acnea, keloid scar, and other inflammatory or allergic conditions of the skin.

In some embodiments, the present invention provides a method for treating an inflammatory disease, disorder, or condition in the vessel/blood. In some embodiments, an inflammatory disease, disorder, or condition in the vessel/blood is selected from Behcet's disease, vasculitis, sepsis, tumor angiogenesis, atherosclerosis, proliferative vascular disease, and restenosis.

In some embodiments, the present invention provides a method for treating an inflammatory disease, disorder, or condition in the eye. In some embodiments, an inflammatory disease, disorder, or condition in the eye is selected from conjunctivitis, scleritis, episcleritis, panuveitis, choroiditis, chorioretinitis, neuroretinitis, uveitis, orbital inflammatory disease, and optical neuritis.

In some embodiments, the present invention provides a method for treating an inflammatory disease, disorder, or condition in the central or peripheral nervous system. In some embodiments, an inflammatory disease, disorder, or condition in the central or peripheral nervous system is selected from non-viral and viral encephalitis and meningitis, depression, neuropathic pain, including chronic pain, traumatic brain injury, including stroke, Alzheimer disease, Parkinson disease, Myelitis, Charcot-Marie-Tooth disease type 1 (including CMT1A and CMT1B), Multiple sclerosis, Amyotrophic lateral sclerosis (ALS), Creutzfeldt-Jakob disease, demyelinating polyneuropathy and peripheral neuropathy.

In some embodiments, the present invention provides a method for treating an autoimmune disease, disorder, or condition. In some embodiments, an autoimmune disease, disorder, or condition is selected from Lupus, including in the skin and kidney, Guillain-Barre syndrome, Myasthenia gravis, Hashimoto's thyroiditis, idiopathic purpura, aplastic anemia, Graves disease, and Myocarditis.

In some embodiments, the present invention provides a method for treating an inflammatory disease, disorder, or condition in the intestine. In some embodiments, an inflammatory disease, disorder, or condition in the intestine is selected from intestinal failure, Ulcerative colitis, and Crohn's disease, eosinophilic esophagitis and inflammation associated with colon carcinoma.

In some embodiments, the present invention provides a method for treating an inflammatory disease, disorder, or condition in the reproductive system. In some embodiments, an inflammatory disease, disorder, or condition in the reproductive system is selected from endometriosis, uterine fibroma, prostate dysplasia or growth, and cervix dysplasia.

In some embodiments, the present invention provides a method for treating an inflammatory disease, disorder, or condition in the bone and/or joints. In some embodiments, an inflammatory disease, disorder, or condition in the bone and/or joints is selected from juvenile idiopathic arthritis, psoriatic arthritis, periodontitis, and hand, foot, ankle, knee, hip, shoulder, elbow or spine arthritis and/or demineralization.

In some embodiment, the present invention provides a method for treating an inflammatory *Coronaviridae* infection and conditions related thereto, in particular wherein the *Coronaviridae* is a Sarbecovirus selected from Severe Acute Respiratory Syndrome-related coronaviruses, even more particularly wherein the Severe Acute Respiratory Syndrome (SARS)-related coronaviruses are selected from the group consisting of: SARS-CoV, SARSr-CoV WIV1, SARSr-CoV HKU3, SARSr-CoV RP3, SARS-CoV-2; including strains responsible for COVID-19 and their mutants.

Advantageously, the patients having, or being at risk of a having a condition related to a *Coronaviridae infection* can also be considered.

According to particular embodiments, the condition related to a *Coronaviridae* infection which are particularly considered include: pulmonary fibrosis, vasculitis, Kawasaki disease and tissue damage or destruction, in particular lung tissue damage and destruction.

As used herein, *"repairing and remodeling tissue"* means promoting healing of tissues that have been damaged or destroyed by a disease, and namely lung tissue devastated by *Coronaviridae infection* or gastrointestinal tissue devasted by *Coronaviridae infection,* at least by not delaying the tissue repair, as usually stated in the framework of treatments with classical anti-inflammatory diseases, as for example corticosteroids which are the best representative of this class of drugs.

Unless instructed otherwise, all the disclosed combinations are specifically considered herein for the treatment or prevention of *Coronaviridae,* which may thus refer indifferently to any member of the said *Coronaviridae* family in the sense of the Baltimore convention.

As used herein, the term "*Coronaviridae* " refers to the corresponding family of RNA viruses belonging to the group IV of the Baltimore classification, which is it iself par of the *Cornidovirineae* suborder and of the *Nidovirales* Order. The *Coronaviridae* family includes both the *Letovirinae* and *Orthocoronavirinae* subfamilies.

As used herein, the term "*Orthocoronavirinae"* refers to the corresponding family of the Baltimore classification, which includes the *Alphacoronavirus, Betacoronavirus, Deltacoronavirus,* and *Gammacoronavirus* genus.

As used herein, the term "*Alphacoronavirus*" refers to the corresponding family of the Baltimore classification, which includes the *Colacovirus, Decacovirus, Duvinacovirus, Luchacovirus, Minacovirus, Minunacovirus, Myotacovirus, Myctacovirus, Pedacovirus, Rhinacovirus, Setracovirus,* and *Tegacovirus* subgenus. In a non-exhaustive manner, this includes the following species: *Bat coronavirus CDPHE15, Bat coronavirus HKU10, Rhinolophus ferrumequinum alphacoronavirus HuB-2013, Human coronavirus 229E, Lucheng Rn rat coronavirus, Ferret coronavirus, Mink coronavirus 1, Miniopterus bat coronavirus 1, Miniopterus bat coronavirus HKU8, Myotis ricketti alphacoronavirus Sax-2011, Nyctalus velutinus alphacoronavirus SC-2013, Porcine epidemic diarrhea virus, Scotophilus bat coronavirus 512, Rhinolophus bat coronavirus HKU2, Human coronavirus NL63, NL63-related bat coronavirus strain BtKYNL63-9b, Alphacoronavirus 1.*

As used herein, the term *"Betacoronavirus"* refers to the corresponding family of the Baltimore classification, which includes the *Embecovirus, Hibecovirus, Merbecovirus, Nobecovirus,* and *Sarbecovirus* subgenus. In a non-exhaustive manner, this includes the following species: *Betacoronavirus 1, China Rattus coronavirus HKU24, Human coronavirus HKU1, Murine coronavirus, Bat Hp-betacoronavirus Zhejiang2013, Hedgehog coronavirus 1, Middle East respiratory syndrome-related coronavirus, Pipistrellus bat coronavirus HKU5, Tylonycteris bat coronavirus HKU4, Hedgehog coronavirus 1, Middle East respiratory syndrome-related coronavirus, Pipistrellus bat coronavirus HKU5, Tylonycteris bat coronavirus HKU4, Rousettus bat coronavirus GCCDC1, Rousettus bat coronavirus HKU9, Severe acute respiratory syndrome-related coronavirus.*

As used herein, the term *"Severe acute respiratory syndrome-related coronavirus",* or SARS virus, includes, in a non-exhaustive manner, the *SARS-CoV, SARSr-CoV WIV1, SARSr-CoV HKU3, SARSr-CoV RP3,* and *SARS-CoV-2; including strains responsible for COVID-19 and their mutants.*

As used herein, the term *"Deltacoronavirus"* refers to the corresponding family of the Baltimore classification, which includes the *Andecovirus, Buldecovirus, Herdecovirus,* and *Moordecovirus* subgenus. In a non-exhaustive manner, this includes the following species: *Wigeon coronavirus HKU20, Bulbul coronavirus HKU11, Coronavirus HKU15, Munia coronavirus HKU13, White-eye coronavirus HKU16, Night heron coronavirus HKU19, Common moorhen coronavirus HKU21.*

As used herein, the term *"Gammacoronavirus"* refers to the corresponding family of the Baltimore classification, which includes the *Cegacovirus* and *Igacovirus* subgenus. In a non-exhaustive manner, this includes the following species: *Beluga whale coronavirus SW1* and *Avian coronavirus.*

According to a particular embodiment, the pharmaceutical composition according to the present invention may be used in the treatment of an inflammatory bowel disease (IBD), including Crohn's disease or ulcerative colitis, dermatitis and rheumatoid arthritis.

According to a particular embodiment, the pharmaceutical composition according to the present invention may be used in the treatment of ulcerative colitis, Crohn's disease and/or eosinophilic esophagitis.

The present invention further relates to a combination of a compound of formula (I) as defined herein after, a pharmaceutically acceptable salt thereof, prodrug thereof or a metabolite thereof, in particular 8-Chloro-N-(4-(trifluoromethoxy)phenyl)quinoline-2-amine, with a S1P receptor modulator, or pharmaceutically acceptable salt thereof, for use in the treatment of inflammatory diseases, disorders or conditions as detailed above in a patient, wherein the S1P receptor modulator is administered in a daily dose reduced by at least 20%, at least 30%, or even at least 40% in comparison to a dose adapted to the treatment of the same inflammatory disease for the same patient, in absence of a compound of formula (I).

The inflammation modulating capacity of pharmaceutical combination according to the present invention can be assessed for Inflammatory bowel disease (IBD) and Rheumatoid Arthritis (RA). The Dextran Sulphate Sodium (DSS-) induced colitis mouse model can be used for studying Inflammatory Bowel Disease (see Perŝe & Cerar; "Dextran Sodium Sulphate Colitis Mouse Model: Traps and Tricks"; Journal of Biomedicine and Biotechnology (2012); 718617). The collagen-induced arthritis model can be used for studying Rheumatoid Arthritis, as shown in Brand et al. ("Collagen-induced arthritis"; Nature Protocols; (2007); 2(5):1269-75).

Indeed, administration of a pharmaceutical combination as defined above leads to an *in vivo* improvement on colon length, weight loss and inflammation in the colon , in DSS- induced colitis mouse models with synergy observed when compared to administration of each of the compounds alone.

The administration of a pharmaceutical combination as defined above leads to a significant decreased joint swelling and lowered signs of inflammation based on a collagen-induced arthritis mouse model with synergy observed for the combination when compared to administration of each of the compounds of the combination alone.

### Pharmaceutical composition, method of treatment and administration scheme

According to another embodiment, the invention provides a pharmaceutical composition comprising a compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite with a S1P receptor modulator or a pharmaceutically acceptable salt thereof and a pharmaceutically acceptable carrier, adjuvant, or vehicle.

The invention further relates to a pharmaceutical kit, in particular intended for treating disorders or conditions as detailed herein above and in particular inflammatory bowel disease, including ulcerative colitis and Crohn's disease rheumatoid arthritis and dermatitis, comprising:
(i) a first galenical formulation comprising a compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine or one of its pharmaceutically acceptable salt or metabolite thereof, and particularly of its glucuronide metabolite, and
(ii) a second galenical formulation comprising a S1P receptor modulator or a pharmaceutically acceptable salt thereof.

According to a particular embodiment, a pharmaceutical composition or a kit according to the invention comprises a compound of formula (I) as defined above being 8-Chloro-N-(4-(trifluoromethoxy)phenyl)quinolin-2-amine or one of its pharmaceutically acceptable salt.

According to a particular embodiment, a pharmaceutical composition or kit according to the invention comprises a S1P receptor modulator selected from fingolimod, ponesimod, siponimod, ozanimod, cenerimod, or etrasimod; or a pharmaceutically acceptable salt thereof, and is in particular etrasimod.

According to a particular embodiment, a pharmaceutical composition or a kit according to the invention comprises 8-Chloro-N-(4-(trifluoromethoxy)phenyl)quinolin-2-amine or one of its pharmaceutically acceptable salt or metabolite thereof and etrasimod or a pharmaceutically acceptable salt thereof.

According to a particular embodiment, a pharmaceutical composition or a kit according to the invention comprises 8-Chloro-N-(4-(trifluoromethoxy)phenyl)quinolin-2-amine and etrasimod.

In certain embodiments, a pharmaceutical composition of this invention is formulated for administration to a patient in need of such composition. In some embodiments, a composition of this invention is formulated for oral administration to a patient.

The term "pharmaceutically acceptable carrier, adjuvant, or vehicle" refers to a non- toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present invention may be administered orally, parenterally, by inhalation spray, topically, rectally, nasally, buccally, vaginally, cutaneous patch or via an implanted reservoir or sustained release oral or parenteral dosage form. The term "parenteral" as used herein includes subcutaneous, intravenous, intraperitoneal, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutical compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically transdermal patches may also be used.

For topical applications, provided pharmaceutical compositions may be formulated in a suitable ointment containing the active component suspended or dissolved in one or more carriers. Carriers for topical administration of combination of this invention include, but are not limited to, mineral oil, liquid petrolatum, white petrolatum, propylene glycol, polyoxyethylene, polyoxypropylene compound, emulsifying wax and water. Alternatively, provided pharmaceutical compositions can be formulated in a suitable lotion or cream containing the active components suspended or dissolved in one or more pharmaceutically acceptable carriers.

Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

Pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. Most preferably, pharmaceutical compositions of this invention are formulated for oral administration. Such formulations may be administered with or without food.

In some embodiments, pharmaceutical compositions of this invention are administered without food. In other embodiments, pharmaceutical compositions of this invention are administered with food.

According to some embodiments, the invention further relates to a method for treating inflammatory diseases, disorders or conditions as detailed herein above and in particular inflammatory bowel disease, including ulcerative colitis and Crohn's disease, rheumatoid arthritis and dermatitis in a patient in need thereof, consisting of administering to a patient in need thereof an effective amount of a compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine and an effective amount of a S1P receptor modulator, or pharmaceutically acceptable salt thereof, in particular etrasimod or one of its pharmaceutically acceptable salts.

### Doses and regimen

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

In one embodiment, the treatment is continuous for only one from the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and S1P receptor modulator, or pharmaceutically acceptable salt thereof or for both or non continuous for only one from the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and S1P receptor modulator, or pharmaceutically acceptable salt thereof or for both.

In one embodiment, the treatment is continuous for both the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and for S1P receptor modulator, or pharmaceutically acceptable salt thereof.

A "continuous treatment" means a long-term treatment, which can be implemented, including with various administration frequencies, preferably twice a day, and more preferably once a day.

Administration of the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and the S1P receptor modulator, or pharmaceutically acceptable salt thereof may be simultaneous, separate or spread out over time.

The combination can be administered repeatedly over the course of several sequences or cycles according to a protocol which depends on the nature of the disease and intensity of the disease to be treated and also on the patient to be treated (age, weight, previous treatment(s), etc.). The protocol can be determined by any specialized practitioner.

Various sequences or cycles of administration respectively of the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and the S1P receptor modulator, or pharmaceutically acceptable salt thereof may be implemented within the framework of the present invention.

According to a particular embodiment, and to one of the possible sequences of administration, the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, is administered to the patient during a first phase of treatment and the patient is then treated with the S1P receptor modulator, or pharmaceutically acceptable salt thereof in a second phase of the treatment or inversely. Said both phases may overlap or not.

Whatever the dose regimen, typically, the dose of the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, may range in human between 1 mg per day and 50 mg per day, in particular between 5 mg per day and 25 mg per day, more particularly between 10 mg per day and 25mg per day.

Whatever the dose regimen, typically, the daily dose of the S1P receptor modulator, or pharmaceutically acceptable salt thereof, in particular etrasimod, may range between 0.1 mg per day and 2 mg per day, in particular between 0.2 mg per day and 2 mg per day, more particularly between 0.5 and 2 mg per day, for example between 0.5 and 1 mg per day or between 0.2 and 1 mg per day.

As an alternative of the regimen as described above, the administration of the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, may be not continuous whereas the administration of the S1P receptor modulator, or pharmaceutically acceptable salt thereof, in particular etrasimod, is continuous or not.

As another alternative of the sequential administration as described above, the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and the S1P receptor modulator, or pharmaceutically acceptable salt thereof, in particular etrasimod, may be administered in a unique dosage form or unit pharmaceutical preparation.

Accordingly, the present invention provides a single unit dosage form comprising the compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, and the S1P receptor modulator, or pharmaceutically acceptable salt thereof, in particular etrasimod, and a pharmaceutically acceptable carrier, adjuvant, or vehicle.

In a particular embodiment the pharmaceutical composition according to the invention, comprises an amount of compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine, ranging from 1 mg and 50 mg, in particular from 5 mg and 25 mg, more particularly from 10 mg and 25mg and an amount of S1P receptor modulator, or pharmaceutically acceptable salt thereof, in particular etrasimod, ranging from 0.1 and 2 mg, in particular between 0.2 and 2 mg, more particularly between 0.5 and 2 mg.

All combinations of doses, frequencies and treatment period are encompassed within the scope of the present invention.

In one embodiment, the treatment is a continuous treatment without induction phase.

In one embodiment, an induction dose is implemented during an induction sequence, which can last 4 to 16 weeks, followed by a further sequence of administration wherein the daily dose of only one of the compounds of the pharmaceutical combination as defined above is reduced by 20% to 60% with respect to the induction daily dose for said compound of the combination, in particular reduced by 30% to 50%.

In an embodiment, herein is provided a pharmaceutical combination as defined above for use as defined above, for treating a patient, wherein a presence and/or expression level of miR-124 in a blood and/or tissue sample of the patient is measured prior to and during the use, in particular for adjusting the dosage of compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine.

In one embodiment, herein is further provided a method of the present invention for treating an inflammatory disease, disorder or condition further comprises measuring and/or monitoring a presence and/or level of a biomarker in a patient. In some embodiments, a presence and/or level of a biomarker is measured in a patient's biological sample. In some embodiments, a patient's biological sample is a blood sample. In some embodiments, a patient's biological sample is a tissue sample. In some embodiments, a biomarker measured and/or monitored in a method of the present invention is miR-124, as described in WO 2014/111892, the entire content of which is incorporated herein by reference. In some embodiments, a method of the present invention for treating an inflammatory disease, disorder or condition further comprises measuring and/or monitoring a presence and/or expression level of miR-124 in a patient prior to administering a compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine. In some embodiments, a method of the present invention for treating an inflammatory disease, disorder or condition further comprises measuring and/or monitoring a presence and/or expression level of miR-124 in a patient during the course of a treatment with a pharmaceutical combination as defined above.

In some embodiments, a method of the present invention for treating an inflammatory disease, disorder or condition further comprises adjusting (such as increasing or decreasing) dosage regimen (such as dose amount and/or dose schedule) of a compound of formula (I) as defined herein above, a pharmaceutically acceptable salt thereof, prodrug thereof or a metabolite thereof, in particular 8-Chloro-N-(4-(trifluoromethoxy)phenyl)quinolin-2-amine to be administered to a patient, by measuring and/or monitoring a presence and/or expression level of miR-124 in the patient.

In an embodiment, herein is provided a pharmaceutical combination as defined above for use as defined above, for treating a patient, wherein white blood cell counts and in particular T cells counts are measured prior to and during the use, in particular for adjusting the dosage of S 1P receptor modulator, or pharmaceutically acceptable salt thereof, in particular etrasimod.

In one embodiment, herein is further provided a method of the present invention for treating an inflammatory disease, disorder or condition further comprises measuring and/or monitoring white blood cell counts in particular T-cells counts in a patient. In some embodiments, white blood cell counts, in particular T-cells counts is measured in a patient's biological sample. In some embodiments, a patient's biological sample is a blood sample. In some embodiments, a patient's biological sample is a tissue sample. In some embodiments, a method of the present invention for treating an inflammatory disease, disorder or condition further comprises measuring white blood cell counts, in particular T-cells counts in a patient prior to administering a S1P receptor modulator, or pharmaceutically acceptable salt thereof, in particular etrasimod. In some embodiments, a method of the present invention for treating an inflammatory disease, disorder or condition further comprises measuring white blood cell counts, in particular T-cells counts in a patient during the course of a treatment with the pharmaceutical combination as defined above.

In some embodiments, a method of the present invention for treating an inflammatory disease, disorder or condition further comprises adjusting (such as increasing or decreasing) dosage regimen (such as dose amount and/or dose schedule) of a S1P receptor modulator, or pharmaceutically acceptable salt thereof, in particular etrasimod to be administered to a patient, by measuring white blood cell counts, in particular T-cells counts in the patient.

In some embodiments, the present invention provides a pharmaceutical composition comprising a combination as described herein and one or more additional therapeutic agents.

Such additional therapeutic agents may be small molecules or recombinant biologic agents and include, for example, acetaminophen, non-steroidal anti-inflammatory drugs (NSAIDS) such as aspirin, ibuprofen, naproxen, etodolac (Lodine^{®}) and celecoxib, colchicine (Colcrys^{®}), corticosteroids such as prednisone, prednisolone, methylprednisolone, hydrocortisone, and the like, probenecid, allopurinol, febuxostat (Uloric^{®}), sulfasalazine (Azulfidine^{®}), antimalarials such as hydroxychloroquine (Plaquenil^{®}) and chloroquine (Aralen^{®}), methotrexate (Rheumatrex^{®}), gold salts such as gold thioglucose (Solganal^{®}), gold thiomalate (Myochrysine^{®}) and auranofin (Ridaura^{®}), D-penicillamine (Depen^{®} or Cuprimine^{®}), azathioprine (Imuran^{®}), cyclophosphamide (Cytoxan^{®}), chlorambucil (Leukeran^{®}), cyclosporine (Sandimmune^{®}, Neoral^{®}), tacrolimus, sirolimus, mycophenolate, leflunomide (Arava^{®}) and "anti-TNF" agents such as etanercept (Enbrel^{®}), infliximab (Remicade^{®}), golimumab (Simponi^{®}), certolizumab pegol (Cimzia^{®}) and adalimumab (Humira^{®}), "anti-IL-1" agents such as anakinra (Kineret^{®}) and rilonacept (Arcalyst^{®}), anti-T cell antibodies such as Thymoglobulin, IV Immunoglobulins (IVIg), canakinumab (Ilaris^{®}), anti-Jak inhibitors such as tofacitinib, upadacitinib, antibodies such as rituximab (Rituxan^{®}), "anti-T-cell" agents such as abatacept (Orencia^{®}), "anti-IL-6" agents such as tocilizumab (Actemra^{®}), diclofenac, cortisone, hyaluronic acid (Synvisc^{®} or Hyalgan^{®}), monoclonal antibodies such as tanezumab, anticoagulants such as heparin (Calcinparine^{®} or Liquaemin^{®}) and warfarin (Coumadin^{®}), antidiarrheals such as diphenoxylate (Lomotil^{®}) and loperamide (Imodium^{®}), bile acid binding agents such as cholestyramine, alosetron (Lotronex^{®}), lubiprostone (Amitiza^{®}), laxatives such as Milk of Magnesia, polyethylene glycol (MiraLax^{®}), Dulcolax^{®}, Correctol^{®} and Senokot^{®}, anticholinergics or antispasmodics such as dicyclomine (Bentyl^{®}), Singulair^{®}, beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), inhaled corticosteroids such as beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), and flunisolide (Aerobid^{®}), Afviar^{®}, Symbicort^{®}, Dulera^{®}, cromolyn sodium (Intal^{®}), methylxanthines such as theophylline (Theo-Dur^{®}, Theolair^{®}, Slo-bid^{®}, Uniphyl^{®}, Theo-24^{®}) and aminophylline, IgE antibodies such as omalizumab (Xolair^{®}), nucleoside reverse transcriptase inhibitors such as zidovudine (Retrovir^{®}), abacavir (Ziagen^{®}), abacavir/lamivudine (Epzicom^{®}), abacavir/lamivudine/zidovudine (Trizivir^{®}), didanosine (Videx^{®}), emtricitabine (Emtriva^{®}), lamivudine (Epivir^{®}), lamivudine/zidovudine (Combivir^{®}), stavudine (Zerit^{®}), and zalcitabine (Hivid^{®}), non-nucleoside reverse transcriptase inhibitors such as delavirdine (Rescriptor^{®}), efavirenz (Sustiva^{®}), nevairapine (Viramune^{®}) and etravirine (Intelence^{®}), nucleotide reverse transcriptase inhibitors such as tenofovir (Viread^{®}), protease inhibitors such as amprenavir (Agenerase^{®}), atazanavir (Reyataz^{®}), darunavir (Prezista^{®}), fosamprenavir (Lexiva^{®}), indinavir (Crixivan^{®}), lopinavir and ritonavir (Kaletra^{®}), nelfinavir (Viracept^{®}), ritonavir (Norvir^{®}), saquinavir (Fortovase^{®} or Invirase^{®}), and tipranavir (Aptivus^{®}), entry inhibitors such as enfuvirtide (Fuzeon^{®}) and maraviroc (Selzentry^{®}), integrase inhibitors such as raltegravir (Isentress^{®}), doxorubicin (Hydrodaunorubicin^{®}), vincristine (Oncovin^{®}), bortezomib (Velcade^{®}), and dexamethasone (Decadron ^{®}) in combination with lenalidomide (Revlimid ^{®}), anti-IL36 agents such as BI655130, Dihydroorotate dehydrogenase inhibitors such as IMU-838, anti-OX40 agents such as KHK-4083, microbiome agents such as RBX2660, SER-287, Narrow spectrum kinase inhibitors such as TOP-1288, anti-CD40 agents such as BI-655064 and FFP-104, guanylate cyclase agonists such as dolcanatide, sphingosine kinase inhibitors such as opaganib, anti-IL-12/IL-23 agents such as AK-101, Ubiquitin protein ligase complex inhibitors such as BBT- 401, sphingosine receptors modulators such as BMS-986166, P38MAPK/PDE4 inhibitors such as CBS-3595, CCR9 antagonists such as CCX-507, FimH antagonists such as EB-8018, HIF-PH inhibitors such as FG-6874, HIF-1α stabilizer such as GB-004, MAP3K8 protein inhibitors such as GS-4875, LAG-3 antibdies such as GSK-2831781, RIP2 kinase inhibitors such as GSK- 2983559, Farnesoid X receptor agonist such as MET-409, CCK2 antagonists such as PNB-001, IL-23 Receptor antagonists such as PTG-200, Purinergic P2X7 receptor antagonists such as SGM-1019, PDE4 inhibiotrs such as Apremilast, ICAM-1 inhibitors such as alicaforsen sodium, Anti- IL23 agents such as guselkumab, brazikumab and mirkizumab, ant-IL-15 agents such as AMG-714, TYK-2 inhibitors such as BMS-986165, NK Cells activators such as CNDO-201, RIP-1 kinase inhibitors such as GSK-2982772, anti-NKGD2 agents such as JNJ-4500, CXCL-10 antibodies such as JT-02, IL-22 receptor agonists such as RG-7880, GATA-3 antagonists such as SB-012 and Colony-stimulating factor-1 receptor inhibitors such as edicotinib or any combination(s) thereof.

Said additional therapeutic agents may alternately be selected from non-steroidal anti-inflammatory drugs (NSAIDS) such as aspirin, ibuprofen, naproxen, etodolac (Lodine^{®}) and celecoxib, colchicine (Colcrys^{®}), corticosteroids such as prednisone, prednisolone, methylprednisolone, hydrocortisone, and the like, probenecid, allopurinol and febuxostat (Uloric^{®}), in particular for the treatment of gout.

In particular for the treatment of rheumatoid arthritis, said additional therapeutic agents may alternately be selected from non-steroidal anti-inflammatory drugs (NSAIDS) such as aspirin, ibuprofen, naproxen, etodolac (Lodine^{®}) and celecoxib, corticosteroids such as prednisone, prednisolone, methylprednisolone, hydrocortisone, and the like, sulfasalazine (Azulfidine^{®}), antimalarials such as hydroxychloroquine (Plaquenil^{®}) and chloroquine (Aralen^{®}), methotrexate (Rheumatrex^{®}), gold salts such as gold thioglucose (Solganal^{®}), gold thiomalate (Myochrysine^{®}) and auranofin (Ridaura^{®}), D-penicillamine (Depen^{®} or Cuprimine^{®}), azathioprine (Imuran^{®}), cyclophosphamide (Cytoxan^{®}), chlorambucil (Leukeran^{®}), cyclosporine (Sandimmune^{®}), leflunomide (Arava^{®}) and "anti-TNF" agents such as etanercept (Enbrel^{®}), infliximab (Remicade^{®}), golimumab (Simponi^{®}), certolizumab pegol (Cimzia^{®}) and adalimumab (Humira^{®}), "anti-IL-1" agents such as anakinra (Kineret^{®}) and rilonacept (Arcalyst^{®}), antibodies such as rituximab (Rituxan^{®}), "anti-T-cell" agents such as abatacept (Orencia^{®}) and "anti-IL-6" agents such as tocilizumab (Actemra^{®}).

In particular for the treatment of osteoarthritis, said additional therapeutic agents may be selected from acetaminophen, non-steroidal anti- inflammatory drugs (NSAIDS) such as aspirin, ibuprofen, naproxen, etodolac (Lodine^{®}) and celecoxib, diclofenac, cortisone, hyaluronic acid (Synvisc^{®} or Hyalgan^{®}) and monoclonal antibodies such as tanezumab.

In particular for the treatment of lupus, said additional therapeutic agents may be selected from acetaminophen, non-steroidal anti-inflammatory drugs (NSAIDS) such as aspirin, ibuprofen, naproxen, etodolac (Lodine^{®}) and celecoxib, corticosteroids such as prednisone, prednisolone, methylprednisolone, hydrocortisone, and the like, antimalarials such as hydroxychloroquine (Plaquenil^{®}) and chloroquine (Aralen^{®}), cyclophosphamide (Cytoxan^{®}), methotrexate (Rheumatrex^{®}), azathioprine (Imuran^{®}) and anticoagulants such as heparin (Calcinparine^{®} or Liquaemin^{®}) and warfarin (Coumadin^{®}).

In particular for the treatment of Crohn's disease, ulcerative colitis, or inflammatory bowel disease, the additional therapeutic agents may be selected from mesalamine (Asacol^{®}) sulfasalazine (Azulfidine^{®}), antidiarrheals such as diphenoxylate (Lomotil^{®}) and loperamide (Imodium^{®}), bile acid binding agents such as cholestyramine, alosetron (Lotronex^{®}), lubiprostone (Amitiza^{®}), laxatives such as Milk of Magnesia, polyethylene glycol (MiraLax^{®}), Dulcolax^{®}, Correctol^{®} and Senokot^{®} and anticholinergics or antispasmodics such as dicyclomine (Bentyl^{®}), anti-TNF therapies, steroids, and antibiotics such as Flagyl or ciprofloxacin.

In particular of the treatment of asthma, the additional therapeutic agents may be selected from Singulair^{®}, beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), inhaled corticosteroids such as prednisone, prednisolone, beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), flunisolide (Aerobid^{®}), Afviar^{®}, Symbicort^{®}, and Dulera^{®}, cromolyn sodium (Intal^{®}), methylxanthines such as theophylline (Theo-Dur^{®}, Theolair^{®}, Slo-bid^{®}, Uniphyl^{®}, Theo-24^{®}) and aminophylline, and IgE antibodies such as omalizumab (Xolair^{®}).

In particular for the treatment of COPD, said additional therapeutic agents may be selected from beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), methylxanthines such as theophylline (Theo-Dur^{®}, Theolair^{®}, Slo-bid^{®}, Uniphyl^{®}, Theo-24^{®}) and aminophylline, inhaled corticosteroids such as prednisone, prednisolone, beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), flunisolide (Aerobid^{®}), Afviar^{®}, Symbicort^{®}, and Dulera^{®}.

In particular for the treatment of organ transplant rejection or graft vs. host disease, said additional therapeutic agents may be selected from a steroid, cyclosporin, FK506, rapamycin, a hedgehog signaling inhibitor, a BTK inhibitor, a JAK/pan-JAK inhibitor, a TYK2 inhibitor, a PI3K inhibitor, and a SYK inhibitor.

In particular for the treatment of *Coronaviridae* infection and conditions related thereto, said additional therapeutic agents may be selected from a dynamin-2 inhibitor, such as Dynasore; an antibiotic, such as one selected from the group consisting of beta-lactams, fluoroquinolones, and macrolides, such as azythromicin; remdesivir; ribavirin; ritonavir; lopanivir; chloroquine or hydroxychloroquine; beta-interferon; an anti-inflammatory compound, such as one selected from the group consisting of: anti-TNF, Jak inhibitors, anti-IL6 antibodies, IL6 receptor antagonists; and a calcium inhibitor such as diltiazem.

The combinations of the invention may be the subject of pharmacological tests which demonstrate their relevance as active combination in therapy and in particular for preventing inflammatory disease.

For both following examples, the combination of the invention is a combination of 8-Chloro-N-(4-(trifluoromethoxy)phenyl)quinolin-2-amine and etrasimod, named "the combination".

### Example 1: Effect of the combination of the invention on the (DSS-) induced colitis model

### A. Material & Methods

### Mouse models

### DSS model

A commonly used mouse model of inflammatory bowel disease is the Dextran Sulphate Sodium (DSS-) induced colitis model. Typical histological changes of acute DSS-colitis are mucin depletion, epithelial degeneration and the eventual destruction of the mucosal barrier which leads to inflammation and colitis.

Five groups of C57B1/6JO1aHsd mice (n=10 per group) females, 9 weeks old receives DSS administration in the drinking water (2.5%) for 7 days followed by 3 days of water only. Weight loss is measured every day, clinical score is calculated every 2 or 3 days and colon length is measured at day 10. One group is treated with the vehicle of with 8-Chloro-N-(4-(trifluoromethoxy)phenyl)quinolin-2-amine. One group is treated with the vehicle of etrasimod. One group of mice is treated with 8-Chloro-N-(4-(trifluoromethoxy)phenyl)quinolin-2-amine alone at doses comprised between 1 and 40 mg/kg/day. One group of mice is treated with etrasimod at doses comprised between 0.1 and 3 mg/kg/day and one group is treated with a combination of 8-Chloro-N-(4-(trifluoromethoxy)phenyl)quinolin-2-amine at doses comprised between 1 and 40 mg/kg/day and etrasimod at doses between 0.1 to 3 mg/kg/day.

### B. Results

Results are established using the combination as described above.

Combination treatment significantly prevents weight loss, clinical score and colon length reduction after 10 days of treatment. Interestingly, when compared to the groups treated with each drug alone, the combination demonstrates a synergistic effect.

### Example 2: Effect of the combination of the invention on the collagen induced arthritis model

### A. Material & Methods

### Mouse models

### Collagen induced Arthritis model:

Groups of 9 to -10-week-old DBA/1 mice are immunized intradermally with bovine collagen type II emulsified in a 1:1 proportion with complete Freund's adjuvant. Mice are challenged 21 days after the first immunization and the phenotypic appearance of arthritis is evaluated by monitoring every second day the thickness of each hind paw *ankle joint* thickness is *measured using a* dial caliper (0- to 10-mm) test *gauge* using a thickness gage.

One group is treated with the vehicle of with 8-Chloro-N-(4-(trifluoromethoxy)phenyl)quinolin-2-amine for two weeks. One group is treated with the vehicle of etrasimod for two weeks. One group of mice is treated with 8-Chloro-N-(4-(trifluoromethoxy)phenyl)quinolin-2-amine alone at doses comprised between 1 and 40 mg/kg/day for two weeks. One group of mice is treated with etrasimod at doses comprised between 0.1 and 3 mg/kg/day and one group is treated with a combination of 8-Chloro-N-(4-(trifluoromethoxy)phenyl)quinolin-2-amine at doses comprised between 1 and 40 mg/kg/day and etrasimod at doses between 0.1 to 3 mg/day.

### B. Results

Combination treatment significantly prevents swelling of the paws after 2 weeks of treatment. Interestingly, when compared to the groups treated with each drug alone, the combination demonstrates a synergistic effect.

Therefore, the result of the tests carried out on the combination of the invention show that said combination may be useful to treat and/or prevent inflammatory diseases as described further above.

## Claims

1. A pharmaceutical combination of a compound of formula (I) or a pharmaceutically acceptable salt thereof, prodrug thereof or a metabolite thereof with a S1P receptor modulator or a pharmaceutically acceptable salt thereof, wherein the compound of formula (I) has the following formula: 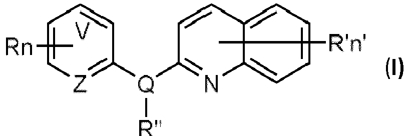 wherein:
Z is C or N;
V is C or N; 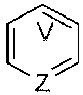 means an aromatic ring wherein V is C or N, and when V is N, V is ortho, meta or para relative to Z;
each R is independently hydrogen, halogen, -CN, hydroxyl, (C₁-C₃)fluoroalkyl, (C₁-C₃)fluoroalkoxy, (C₃-C₆)cycloalkyl, -NO₂, -NR₁R₂, (C₁-C₄)alkoxy, phenoxy, -NR₁-SO₂-NR₁R₂, -NR₁-SO₂-R1, -NR₁-C(=O)-R₁, -NR₁-C(=O)-NR₁R₂, -SO₂-NR₁R₂, -SO₃H, -O-SO₂-OR₃, -O- P(=O)-(OR₃)(OR₄),
-O-CH₂-COOR₃, (C₁-C₃)alkyl, said alkyl being optionally mono- or di-substituted by a hydroxyl group, a group of formula (IIa):
or a group of formula 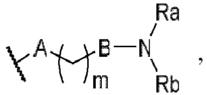 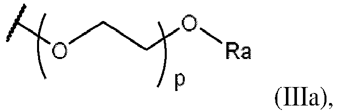
Q is N or O, provided that R" does not exist when Q is O;
each of R₁ and R₂ is independently hydrogen or (C₁-C₃)alkyl;
each of R₃ and R₄ is independently hydrogen, Li⁺, Na⁺, K⁺, N⁺(Ra)₄ or benzyl; n is 1, 2 or 3;
n' is 1, 2 or 3;
each R' is independently hydrogen, (C₁-C₃)alkyl, hydroxyl, halogen, -NO₂, -NR₁R₂, morpholinyl, morpholino, N-methylpiperazinyl, (C₁-C₃)fluoroalkyl, (C₁-C₄)alkoxy,-O-P(=O)-(OR₃)(OR₄),- CN,
a group of formula (IIa): 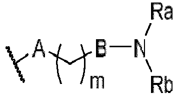
or a group of formula (IIIa): 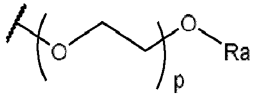
A is a covalent bond, oxygen, or NH;
B is a covalent bond or NH;
m is 1, 2, 3, 4 or 5;
p is 1, 2 or 3; each of Ra and Rb is independently hydrogen, (C₁-C₅)alkyl, or (C₃-C₆)cycloalkyl, or
Ra and Rb form together with the nitrogen atom to which they are attached a saturated 5- or 6- membered heterocycle, said heterocycle being optionally substituted by one or more Ra, provided that when R' is a group (IIa) or (IIIa), n' may be 2 or 3 only if other R' groups are different from said group (IIa) or (IIIa); and
R" is hydrogen, (C₁-C₄)alkyl, or a group of formula (IIa) as defined herein.

2. A pharmaceutical combination according to claim 1, wherein the compound of formula (I) is a compound of formula (Ib): 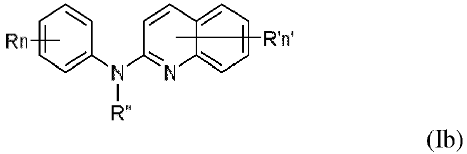 or anyone of its metabolites or a pharmaceutically acceptable salt thereof or prodrug thereof wherein R, R' and R" are as defined in claim 1.

3. A pharmaceutical combination according to claim 1 or 2, wherein the compound of formula (I) is selected from 8-Chloro-N-(4-(trifluoromethoxy)phenyl)quinolin-2-amine or one of its pharmaceutically acceptable salt or metabolite thereof.

4. A pharmaceutical combination according to claim 1, wherein is comprises a metabolite of the compound of formula (I), in particular a glucuronide metabolite thereof.

5. A pharmaceutical combination according to the preceding claim, wherein it comprises the glucuronide metabolite of formula

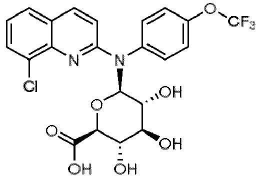

6. A pharmaceutical combination according to anyone of the preceding claims, wherein the S1P receptor modulator is selected from ponesimod, cenerimod, siponimod, fingolimod, ozanimod, or etrasimod, and is in particular etrasimod, and pharmaceutically acceptable salts thereof.

7. A pharmaceutical combination according to anyone of the preceding claims, wherein the compound of formula (I) is 8-Chloro-N-(4-(trifluoromethoxy)phenyl)quinolin-2-amine and the S1P receptor modulator is etrasimod.

8. A pharmaceutical composition comprising a compound of formula (I) as defined in claim 1, a pharmaceutically acceptable salt thereof, prodrug thereof or a metabolite thereof and a S1P receptor modulator or a pharmaceutically acceptable salt thereof and at least one pharmaceutically acceptable excipient.

9. A pharmaceutical composition according to the preceding claim, wherein the compound of formula (I) and the S1P receptor modulator are as defined in anyone of claims 2 to 7.

10. A pharmaceutical composition according to claims 8 or 9, wherein it comprises 8-Chloro-N-(4-(trifluoromethoxy)phenyl)quinolin-2-amine or one of its pharmaceutically acceptable salt and etrasimod or a pharmaceutically acceptable salt thereof and at least one pharmaceutically acceptable excipient.

11. A combination as defined in anyone of claims 1 to 7 for use in for use in the treatment of inflammatory diseases, disorders or conditions as detailed herein after
(a) an inflammatory disease, disorder, or condition in the pancreas selected from diabetes type-1, diabetes type-2, acute and chronic pancreatitis;
(b) an inflammatory disease, disorder, or condition in the kidney selected from glomerulosclerosis, glomerulonephritis, nephritis, acute kidney injury, Berger's disease, Goodpasture's syndrome, Wegener's granulomatosis and kidney transplant acute or chronic rejection;
(c) an inflammatory disease, disorder, or condition in the liver selected from nonalcoholic steatohepatitis (NASH), non-alcoholic fatty liver disease (NAFLD), cholestatic liver disease, sclerosing cholangitis and liver transplant acute or chronic rejection;
(d) an inflammatory disease, disorder, or condition in the lung or heart selected from bronchitis, asthma, chronic obstructive pulmonary disease (COPD), pulmonary fibrosis, pulmonary hypertension, sarcoidosis, myocarditis, pericarditis and lung or heart transplant acute or chronic rejection, *Coronaviridae* infection and conditions related thereto, in particular wherein the *Coronaviridae* is a Sarbecovirus selected from Severe Acute Respiratory Syndrome-related coronaviruses, even more particularly wherein the Severe Acute Respiratory Syndrome (SARS)-related coronaviruses are selected from the group consisting of: SARS-CoV, SARSr-CoV WIV1, SARSr-CoV HKU3, SARSr-CoV RP3, SARS-CoV-2; including including strains responsible for COVID-19 and their mutants;
(e) an inflammatory disease, disorder, or condition in the skin selected from psoriasis, dermatitis, such as eczema, contact dermatitits, atopic dermatitis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acnea, keloid scar, and other inflammatory or allergic conditions of the skin;
(e) an inflammatory disease, disorder, or condition in the vessel/blood selected from Behcet's disease, vasculitis, sepsis, tumor angiogenesis, proliferative vascular disease and restenosis, atherosclerosis;
(h) an inflammatory disease, disorder, or condition in the eye selected from conjunctivitis, scleritis, episcleritis, panuveitis, choroiditis, chorioretinitis, neuroretinitis, uveitis, orbital inflammatory disease, and optical neuritis;
(i) an inflammatory disease, disorder, or condition in the central or peripheral nervous system selected from non-viral and viral encephalitis and meningitis, depression, neuropathic pain, including chronic pain, traumatic brain injury, including stroke, neurodegenerative diseases such as Alzheimer's disease, and Parkinson disease, Myelitis, Charcot-Marie-Tooth disease type 1 (including CMT1A and CMT1B), Amyotrophic lateral sclerosis (ALS), Creutzfeldt-Jakob disease, demyelinating polyneuropathy and peripheral neuropathy;
(i) an autoimmune disease, disorder, or condition selected from Sjogren's syndrome, Lupus, including in the skin and kidney, Guillain-Barre syndrome, Myasthenia gravis, Hashimoto's thyroiditis, idiopathic purpura, aplastic anemia, Graves disease, and Myocarditis;
(k) an inflammatory disease, disorder, or condition in the reproductive system selected from endometriosis, uterine fibroma, prostate dysplasia or growth, and cervix dysplasia;
(1) an inflammatory disease, disorder, or condition in the bone and/or joints selected from rheumatoid arthritis (RA), osteoarthritis (OA), ankylosing spondylitis, juvenile idiopathic arthritis, psoriatic arthritis, periodontitis, and hand, foot, ankle, knee, hip, shoulder, elbow or spine arthritis and/or demineralization;
(m) an inflammatory disease, disorder, or condition in the digestive tract selected from inflammation associated with colon carcinoma, Inflammatory Bowel Disease, including Crohn's disease, Ulcerative Colitis and eosinophilic esophagitis; and
(n) an inflammatory disease, disorder, or condition in the central nervous system selected from Multiple sclerosis (MS), relapsing-remitting multiple sclerosis (RRMS); relapsing forms of multiple sclerosis (RMS) and secondary progressive multiple sclerosis (SPMS).

12. A combination for use according to the preceding claim, wherein the inflammatory disease is inflammatory bowel disease, including ulcerative colitis and Crohn's disease, rheumatoid arthritis and dermatitis.

13. A pharmaceutical combination of a compound of formula (I) as defined in anyone of claims 1 to 5, in particular 8-Chloro-N-(4-(trifluoromethoxy)phenyl)quinolin-2-amine or one of its pharmaceutically acceptable salt, prodrug or metabolite thereof and of a S1P receptor modulator or a pharmaceutically acceptable salt thereof for separate administration, administration spread out over time or simultaneous administration to patients suffering from inflammatory diseases, disorders or conditions, in particular as defined in claim 11.

14. A pharmaceutical combination for separate administration, administration spread out over time or simultaneous administration to patients suffering from inflammatory diseases, disorders or conditions, wherein said and inflammatory diseases, disorders or conditions is inflammatory bowel disease, including ulcerative colitis and Crohn's disease, rheumatoid arthritis and dermatitis.

15. A pharmaceutical kit, in particular intended for treating inflammatory diseases, disorders or conditions as detailed in claim 11, and in particular inflammatory bowel disease, including ulcerative colitis and Crohn's disease, rheumatoid arthritis and dermatitis, comprising:
(i) a first galenical formulation comprising a compound of formula (I) as defined in anyone of claims 1 to 5, in particular 8-Chloro-N-(4-(trifluoromethoxy)phenyl)quinolin-2-amine or one of its pharmaceutically acceptable salt, prodrug or metabolite thereof, and particularly of its glucuronide metabolite as defined in claim 5, and
(ii) a second galenical formulation comprising a S1P receptor modulator or a pharmaceutically acceptable salt thereof, in particular etrasimod or any of its pharmaceutically acceptable salt.
